# EUROPEAN PATENT APPLICATION

(11) **EP 1 514 596 A2**
(43) Date of publication of application: **16.03.2005**
(21) Application number: 04021477.7
(22) Date of filing: 09.09.2004
(51) Int. Cl.: B01J 19/00, B01L 3/00

(54) **Device substrate, method of manufacturing the same, and microchip**

(30) Priority: 11.09.2003 JP 2003320010
(71) Applicant: Matsushita Electric Industrial Co., Ltd., Kadoma-shi, Osaka 571-8501 (JP)
(72) Inventor: Mino, Norihisa, Osaka-shi Osaka (JP)
(74) Representative: Leson, Thomas Johannes Alois, Dipl.-Ing.

(57) **Abstract**

A device substrate comprising a substrate having a flow channel of a liquid, wherein an internal surface of said flow channel has an area non-compatible with said liquid; said area non-compatible with the liquid is formed by a monolayer non-compatible with said liquid; and said monolayer is connected to said substrate via a covalent bond, which allows smooth flow of the liquid in the minute flow channel.

## Description

### Background of the Invention

### Field of the Invention;

The present invention relates to a device substrate having a flow channel and a method of manufacturing the same, in particular to a device substrate for use in analysis of DNAs, proteins, and the like and a microchip comprising the device substrate.

### Description of the related art:

A variety of DNA chips and protein chips have been commercialized as new tools for gene analysis and protein analysis for the purpose of prevention and treatment of diseases, development of new drugs, and others. More recently, developments of so-called integrated microchips were in progress for the purpose of performing more complicated reactions on a single chip, and a known example of such microchips is the "Labchip™".

These integrated microchips commonly have on the substrate, a sample inlet unit, storing units for storing reagents and solvents, flow channels for transporting the samples, reaction solutions, and the like, wastewater reservoirs for storing reaction solutions after analysis, and the like, which are arranged like electric circuits. The sample, reagents, and the like are transported via the flow channels by capillary phenomenon or by control of applied voltage or pressure.

More specifically, a series of procedures, wherein, for example, a sample, solvents and reagents are transported, mixed and reacted through respective flow channels; the reaction solution is analyzed at a particular site; and then the reaction solution is fed via a flow channel into a wastewater reservoir, is accomplished on a single chip. Namely, various functions such as injection, separation, aliquotting, reaction, pump, valve, and the like are accumulated on a single chip.

On such integrated microchip chips, it is possible to perform multi-step reactions using a particular sample, and for example, to perform DNA analysis by extracting and amplifying DNAs with a small amount of sample in a very small area (e.g., Jpn. Unexamined Patent Publication Nos. 2001-513882, 10-148628, 2002-236131, 2002-214175, and 2002-218998).

However, flow channels formed on the integrated microchips described above have a very minute structure, generating the problem that it is quite difficult to mix various kinds of liquids efficiently and uniformly due to the turbulence in flow generated therein.

The integrated microchips also carry the problems that the liquids flowing through the flow channels adhere to the walls of the grooves formed on the substrate due to surface tension or the like and that the reagents and samples in the liquids adhere to the rugged wall surface of the grooves.

As a result, it is difficult to carry out uniform reaction on the microchips, often affecting the analytical accuracy. Further, it is difficult to transport all liquids thoroughly to the terminals of the microchip, i.e., to the areas of analytical units, wastewater reservoirs; and the like, because of the very minute structure of the substrate.

### Brief Summary of Invention

The present invention has been achieved to solve the problems above and an object thereof is to provide a device substrate for use in the microchips whereon a liquid can be transported smoothly through minute flow channel, and a method of manufacturing the same.

To achieve the object, the device substrate according to the present invention is a substrate having a flow channel of a liquid, wherein the substrate has an area non-compatible with the liquid (hereinafter, referred to as "non-compatible area") on the internal surface of the flow channel; the non-compatible area is formed with a monolayer non-compatible with the liquid on the internal surface of the flow channel; and the monolayer is bound to the internal surface via a covalent bond.

The method of manufacturing the device substrate having a flow channel formed thereon according to the present invention comprises a step of forming an area non-compatible with the liquid by: providing a substrate having an active hydrogen on a surface of the substrate in the flow channel; preparing an organic molecule containing a bonding functional group capable of forming a covalent bond with the active hydrogen on the substrate surface at a terminal and a terminal group non-compatible with the liquid at the other terminal; contacting the organic molecule with the substrate surface having the active hydrogen on the substrate; and connecting a monolayer non-compatible with the liquid to the substrate surface by forming a covalent bond in a reaction of the terminal bonding functional group of the organic molecule and the active hydrogen of the substrate.

In the present invention, the "monolayer" is a layer connected to the substrate via a covalent bond. The monolayer according to the present invention may be a single layer (onae layer) or a laminated layer consisting of multiple layers, if the area non-compatible with the liquid can be formed.

The monolayer according to the present invention may be a single kind of layer or a combination of multiple monolayers different in structure, shape, dimension, non-compatibility, or the like. The samples and reagents above may be liquids per se.

### Brief Description of Drawings

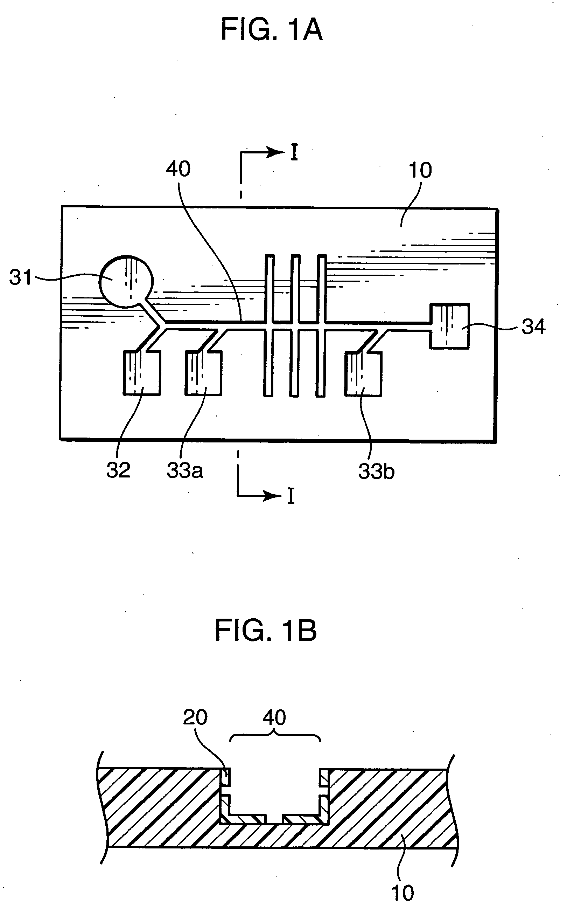

Figures 1A and 1B are schematic views illustrating an example of the microchip according to the present invention. Figure 1A is a plane view of the microchip, and Figure 1B is a cross sectional view thereof.

### Detailed Description of Invention

In the present invention, the structure of the flow channel is not particularly limited, if liquid can flow through, and the flow channel may be, for example, groove, dent, empty hole, hollow, or the like. In addition, the flow channel may be pipe in shape, or the device per se may have a shape wherein a plurality of pipes, i.e., flow channels, are connected to each other.

In the present invention, the monolayer non-compatible with the liquid is preferably, for example, "hydrophobic" if the liquid is aqueous and "lipophobic" if the liquid is an oil-based solvent. The present invention may also be applied to the case when a liquid passing through the flow channel contains a compound having a terminal bonding functional group described below in a similar manner to the organic compound constituting the monolayer. For example, if a monolayer is previously formed on the substrate surface in the flow channel so that the surface becomes non-compatible with the compound, it is possible to prevent covalent bond formation between the active hydrogen of substrate described below and the compound even when a solution containing the organic molecule passes through the flow channel.

In the device substrate according to the present invention, all area of the internal surface of flow channels may be the non-compatible area. Alternatively, the device substrate may have a local area compatible with the liquid (hereinafter, referred to as "compatible area") in the flow channel. Yet alternatively, it is possible to make all internal surface of the some flow channels non-compatible and that of the other channels compatible.

As the structure wherein all internal surfaces of flow channels are non-compatible may be considered as an extension of the structure wherein only part of the internal surface thereof is non-compatible, the structure wherein non-compatible area is formed only locally on the internal surface will be described below.

Substrates having non-compatible areas locally on the internal surface of flow channels includes those having non-compatible areas scattered throughout the entire flow channels from one end to the other and those having non-compatible areas continuously throughout and in parts of the flow channels along the traveling direction of liquids.

An example thereof is a substrate wherein the substrate surface in flow channels is compatible with the liquid and the non-compatible areas are formed continuously along the traveling direction of the liquid on parts of the substrate surface in the flow channel. The compatible areas may be the substrate surface itself, or the compatible areas may be formed by treating the substrate surface in the flow channel in some way.

In an embodiment of the present invention, the non-compatible areas are areas formed by connecting a monolayer non-compatible with the liquid via covalent bonds to the substrate surface in the flow channel.

Such device substrates having a structure wherein compatible areas are formed locally allows faster movement of the liquid in the flow channel and more uniform mixing of samples and reagents in the flow channel.

In other words, if most of the inner surface of the flow channels is made non-compatible and the remaining inner surface compatible, the liquid in the flow channel becomes in contact only with the compatible areas. As a result the compatible areas serve as a guide for feeding the liquid. Such areas, for example, prevent adhesion or retention of the liquid to the internal wall of flow channels and enable faster movement thereof through the channels. As the layer formed thereon is a monolayer having a nano-order thickness, there is practically no difference in level between the non-compatible area whereon the monolayer is formed and the compatible area whereon no monolayer is formed.

An example of the device substrates having the structure above will be described with reference to Figures 1A and 1B. Figure 1A is a schematic plane view of a device substrate, and Figure 1B is a cross sectional view of the device shown in Figure 1A in the I-I direction. Both drawings illustrating a microchip are schematic views provided only for the purpose of describing the present invention in a simpler way, and the dimension and shape therein do not reflect those of an actual microchip.

The device substrate includes a substrate 10 compatible with the liquids, and substrate 10 comprises sample inlet unit 31, solvent unit 32, reagent units 33a and 33b, dent serving as a wastewater reservoir 34, and flow channels (grooves) 40 for connecting the respective dents. As shown in Figure 1B, monolayers 20 non-compatible with the liquid are formed on the internal surface of flow channel 40 along the traveling direction of the liquid. More specifically, the monolayers 20 are formed all over the internal surface of the groove except parts of the bottom and side faces thereof and the areas whereon no monolayer 20 is formed are areas compatible with the liquid. For example, substrates having active hydrogen on the substrate surface in flow channels are preferably used as the compatible substrate. Although there is a difference in level between the areas whereon the monolayer 20 is formed and not formed in the groove of the substrate shown in Figure 1B, the thickness of the monolayer is at the nano order and thus there is practically no difference in level that may affect the transportation of liquid.

The width of the compatible area varies according to the kind of flowing liquid, but is preferably 0.5 µm or more and more preferably 1 µm or more. On the other hand considering the ratio of compatible to non-compatible areas, the width is preferably 10 µm or less and more preferably 5 µm or less.

When most of the internal surfaces of grooves are made non-compatible and only a part thereof compatible along the traveling direction of the liquids in this manner, the liquids become in contact only with the compatible areas during transport of the liquids through the groove. Therefore, the liquids travel rapidly through the flow channels, guided by the compatible areas. In addition, presence of the non-compatible monolayer prevents retention of the liquids on the internal surface of the grooves. Further, tight bonding between the monolayer and the substrate surface via the covalent bonds described below eliminates the problem of separation of the monolayer.

In another embodiment of the flow channel different from that described above, it is possible to produce flow channels wherein liquids flow slower in compatible areas and faster in non-compatible areas, by forming the non-compatible areas scattered along the flow channel from one end to the other.

It is desirable to control the flow rate of liquids in respective flow channels, as the desirable flow rate of the liquids varies according to the kinds of processing in reagent-supplying unit, reaction unit, analytical unit, and the like, but it is extremely difficult to control the flow rate on integrated microchips wherein all these steps are accumulated on a single chip.

By forming non-compatible areas at some parts of continuous flow channels and compatible areas at the other part thereof, it is also possible to change the flow rate of liquids flowing through the flow channels locally, as the liquids flow more rapidly than in compatible areas due to the decrease in the wettability of the liquids flowing through the flow channels in the non-compatible areas.

Additionally, it is possible to modify the degree of non-compatibility by changing the kind of the organic molecule used for forming the monolayer and thus to provide different flow rates at the different sites in non-compatible area.

In this manner, the present invention advantageously provides a device substrate whereon various flow channels different in the flow characteristics of liquids can be formed on the device substrate described above.

Further, it is also possible to control the flow of liquids more accurately, by forming flow channels having a structure in combination of the structures described above.

The dimension of the entire device substrate is not particularly limited, but usually 5 to 100 mm in length, 5 to 100 mm in width, and 500 to 5,000 µm in thickness. The width of the flow channels on the substrate is not particularly limited, but usually 10 µm or more and more preferably 50 µm or more. On the other hand the width is preferably 300 µm or less and more preferably 100 µm or less for miniaturization of the substrate. The depth of the flow channels is preferably 10 µm or more and 100 µm or less, and more preferably 30 µm or more and 50 µm or less, for the same reason. Further, the ratio (area %) of the compatible areas in the entire internal surface of the flow channels is, for example, in the range of about 0.1 to 1%.

In the device substrate according to the present invention, the monolayer may be a single layer or laminated layers consisting of several monolayers. The thickness of the monolayer is, for example, in the range of 0.5 to 2 nm; the thickness of the laminated layers may vary according to the number of layers, but is, for example, in the range of 1 to 100 nm, preferably in the range of 1 to 10 nm, and more preferably in the range of 1 to 6 nm; and the number of layers is, for example, in the range of 2 to 100, preferably in the range of 2 to 50, and particularly preferably in the range of 2 to 6.

Examples of the substrates for use in the present invention are substrates hitherto known in the art including glass substrates, quartz substrates, synthetic quartz substrates, silicon substrates, various polymer substrates such as acrylic substrate, polystyrene substrate, polyvinyl chloride substrate, epoxy resin substrate, silicone resin (polydimethylsilicone) substrate, PMMA (polymethyl methacrylate) substrate, and polycarbonate substrate; ceramic substrates; metal substrates; and the like. Among them, glass and quartz substrates are preferable, as they have a structure having many hydroxyl groups on the surface thereof. The substrates for microchips include those having another substrate adhered or bonded to the substrate described above.

The substrate having in the internal surfaces of flow channels the areas non-compatible with the liquid flowing through the flow channels is prepared by coating the monolayer non-compatible with the liquid on the surface of the substrate having particular grooves.

Hereinafter, the monolayer according to the present invention will be described.

For forming a monolayer, a substrate containing a characteristic group having an active hydrogen such as -OH, -NH₂, =N-H, quaternary ammonium ion, -PO₃H, -SO₃H, -SH, or the like, which is reactive with an organic molecule, are used as the substrate in the present invention. Needless to add, the areas having the active hydrogens may be only on the substrate surface in flow channels whereon the monolayer is desirably formed. The monolayer is preferably prepared in the monolayer-forming process of providing as the organic molecule an organic molecule having a functional group capable of forming a covalent bond in reaction with the active hydrogen on the substrate surface at one end of the molecular chain and a characteristic group non-compatible with the liquid to be placed at the other end, bringing the organic molecule into contact with the substrate, and thus progressing a condensation reaction.

In the substrate above, the portion of organic compound other than the active hydrogen may be present on the substrate. For example, the portion of organic compound other than the active hydrogen may be present inside the substrate, and the portion of organic compound other than the active hydrogen may bind to the constituent elements of the substrate. More specifically, if a substrate has, for example, a metal oxide as the constituent material and the group having an active hydrogen is -PO₃H, all of the -PO₃H group may be exposed or only -OH group of the -PO₃H group may be exposed. The -PO₂- portions hidden in the substrate may be -PO₂- per se, or the oxygen bound to P may bind, for example, to a metal atom (metal ion) M¹ in bulk metal oxide, forming a structure of -P-O-M¹-.

The substrate should have active hydrogens only when a monolayer is formed, and thus may have previously a sufficient amount of active hydrogens for forming a monolayer or the substrate surface in the flow channel may be granted with the active hydrogens before the monolayer-forming process.

The covalent bond formed by the condensation reaction of the active hydrogen with the terminal functional group of an organic molecule described below is at least one covalent bond selected from the group consisting of M-O, M-N and M-S bonds (M: Si, Ti, Al or Sn), depending on the structure of the characteristic group having the active hydrogen present on the substrate and the kind of the organic molecule, i.e., the raw material for monolayer. The bond preferably is preferably a bond containing at least one structure selected from the group consisting of Si-O, Si-N, and Si-S bonds, more preferably a Si-O or Si-N bond, and still more preferably a Si-O bond, from the viewpoint of easier manufacture.

As described above, in the present invention, the organic molecule forming the monolayer has a terminal functional group capable of forming a covalent bond with the substrate surface above at a terminal thereof and a characteristic group non-compatible with the liquid at the other terminal. The characteristic group "non-compatible with the liquid" may be selected suitably according to the kind of liquid used, but is preferably, for example, hydrophobic if the solution is aqueous as in the case where the substrate is used as an integrated microchip.

When an organic molecule of the present invention has a branched structure, the terminal group is one of the terminal groups.

The degree of hydrophobicity is determined relatively according to the liquid to be placed. For example, if the liquid is a solution mainly containing water, the critical surface energy of the substrate surface is preferably 25 mN/m or less and more preferably 8 mN/m or more at 20°C.

The critical surface energy is obtained by measuring contact angles by using standard solutions for measuring critical surface energy and a static contact-angle meter, and is a value determined by plotting the energy values of the standard solutions against the values of cosine contact angles, and extrapolating the line to the energy value at a cosine value of 0.

In addition, the difference in critical surface energy between the hydrophilic and hydrophobic portions when the liquid is an aqueous solution may be selected properly according to the area whereon the monolayer is formed and the aqueous solution used, but is preferably 20 mN/m or more and more preferably 40 mN/m or more. On the other hand if the monolayer is formed with an organic molecule described above, the aforementioned difference is preferably 75 mN/m or less and more preferably 65 mN/m or less.

The degree of hydrophobicity is such that when a droplet of 5.3 µL is dropped on the surface of a monolayer at 20°C, the contact angle between the droplet and the surface is preferably 80 to 180°, more preferably 90 to 180°, and still more preferably 100 to 160°. The contact angle may be determined, for example, according to the measuring method specified in JIS R3257: 1999.

The organic molecule used for forming the hydrophobic monolayer according to the present invention is preferably a molecule having one of the structures represented by the following Formulae (i) to (iii).

Organic molecule (i):
having
   (a) a functional group represented by the following General Formula (1) as the terminal bonding functional group capable of forming a covalent bond with the substrate surface in the flow channel: 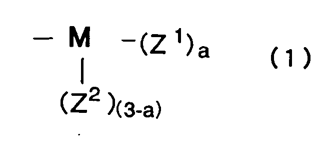 [in General Formula (1), M is Si, Ti, Al or Sn; Z¹ represents at least one atom or atomic group selected from the group consisting of F, Cl, Br, I, -OH, -SCN, -NCO, and alkoxy group having 1 to 5 carbons; Z² represents at least one atom or atomic group selected from the group consisting of H and alkyl group having 1 to 5 carbons; and a is an integer of 1 to 3];
   (b) at least one characteristic group selected from the group consisting of methyl group, halogen-substituted methyl group, vinyl group, cyclic ether group having 2 to 4 carbons, phenyl group, halogen-substituted phenyl group, cyano group and the derivatives thereof as the terminal group not binding to the substrate surface; and
   (c) a bivalent characteristic group represented by the following General Formula (2) between the two terminal groups:

      -C_{b}E_{2b}- (2)

      [in General Formula (2), E represents at least one atom selected from the group consisting of H and F; and b is an integer of 2 to 22].

Organic molecule (ii):
having
   (d) Si as the M represented by General Formula (1); and
   (e) additionally at least one bivalent characteristic group selected from the group consisting of the characteristic groups represented by the following General Formula (3), -O-, -COO-, -C₆H₄- and the derivatives thereof between the carbons of the bivalent characteristic group represented by General Formula (2): 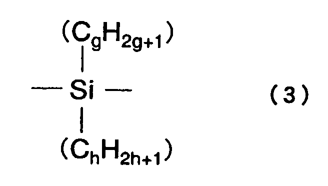 (wherein, g and h in the following General Formula (3) each represent independently an integer of 1 to 3).

Organic molecule (iii):
having
   (f) a functional group represented by the following General Formula (4) as the terminal functional group forming a covalent bond with the substrate surface in the flow channel;
   (g) two monovalent groups selected from the group consisting of methyl group, halogen-substituted methyl group, vinyl group, cyclic ether group having 2 to 4 carbons, phenyl group, halogen-substituted phenyl group, cyano group and the derivatives thereof as the terminal characteristic group not forming a covalent bond therewith; and
   (h) additionally a trivalent characteristic group represented by the following General Formula (5) between the two terminal groups. 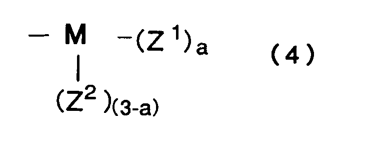 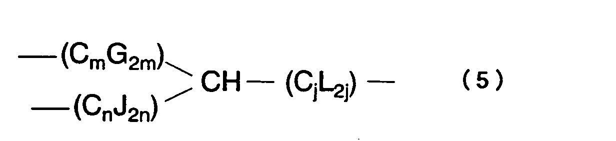
[General Formula (4) is identical with General Formula (1) of organic molecule (i) above; in General Formula (5), CⱼL₂ⱼ is a characteristic group binding to the covalent-bonding terminal functional group; CₘG₂ₘ and CₙJ₂ₙ are characteristic groups binding to the terminal groups not forming a covalent bond; G, J and L may be the same or different from each other and each represent at least one atom selected from the group consisting of H and F; j is an integer of 1 to 18; and m and n each are independently an integer of 0 to 7.].

In the organic molecules having the structures, examples of the covalent-bonding terminal functional groups represented by (1) above include halogenated silyl groups, alkoxysilyl groups, isocyanate silyl groups, alkoxy aluminum groups, halogenated titanium groups, halogenated tin groups, and the like. It is particularly favorable if a=3, and preferable examples thereof include trihalogenated silyl groups, trialkoxysilyl groups, and triisocyanate silyl group.

The halogen atom in the trihalogenated silyl groups is F, Cl, Br, or I. Among trihalogenated silyl groups, chlorosilyl group is preferable. The alkoxy group in the trialkoxy silyl group above is particularly preferably a group having 1 to 3 carbons. Specific examples thereof include methoxysilyl, ethoxysilyl, and butoxysilyl groups.

Organic silane compounds having one of the many substituted silyl groups at one of the terminals form a covalent bond with the substrate as described above, and the monolayers formed are more tightly bound to the substrate. Specifically, an organic molecule is covalently bound to the substrate via a siloxane bond (-Si-O-), by a dehydrohalogenation reaction if the organic molecule is a halogenated silyl group; by a dealcoholization reaction if an alkoxy silyl group; or by an isocyanate-removing reaction if an isocyanate silyl group. The covalent bond formed between the organic molecule and the substrate varies according to the kind of the group having an active hydrogen on the substrate surface, and when the group having an active hydrogen is, for example, an -NH group, -SiN bond is formed as the covalent bond.

Additionally, if the terminal bonding functional group is a multiply substituted silyl group, the silyl group forms covalent bonds at two sites or more, by forming covalent bonds by condensation reaction with the active hydrogen of the substrate not only at one substituent but also at the other substituents as shown in the following General Formula (6). If there are not a sufficient amount of bond-forming active hydrogens on the substrate surface, neighboring organic molecules may bind to each other. 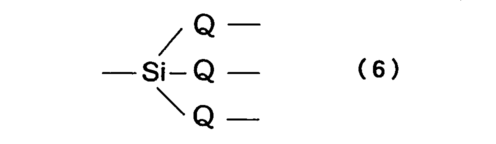 [in the Formula, Q is at least one atom selected from O, N and S; and the Si is covalently bound via each element to the substrate or a neighboring organic silane group.]

In the organic molecule above, the bivalent characteristic group of General Formula (2) or the trivalent characteristic group of General Formula (5) preferably has a main chain having a total carbon number of 8 or more and 22 or less and preferably 8 or more and 18 or less, especially if the substrate is to be used in analytical instruments for analyzing biomacromolecules.

Presence of an intermediate characteristic group having such a total carbon number forces the molecule of the monolayer to orient itself vertical to the substrate, placing the terminal hydrophobic noncovalent-bonding characteristic group at the extreme surface and increasing the difference in hydrophilicity. In a similar manner to ordinary organic compounds, the main chain means a longer chain having the greatest number of carbons if there is a branched chain in the molecule.

Among the intermediate characteristic groups described above, C₂H₄O group is preferable as the cyclic ether group having 2 to 4 carbons. If the organic compound has a C₂H₄O group, the thickness of monolayer can be increased easily by using the ring-opening (addition) reaction of the epoxy groups. A sufficient uniformity in layer thickness can also be achieved easily at the same time.

For example, if the organic compound has a C₂H₄O group at the terminal end not forming a covalent bond, it is possible to expand the thickness of the monolayer, by bringing an alcohol additionally to the monolayer formed, allowing the ring-opening (addition) reaction of the epoxy group to proceed, and thus connecting the portion of the alcohol other than -OH (hydrocarbon group) thereto.

Among the terminal characteristic groups not forming a covalent bond in the organic silane compound described above, CF₃-, CH₂Br-, and CH₂Cl- are preferable, and CF₃- is more preferable as the halogen-substituted methyl group, for obtaining a sufficiently hydrophobic monolayer more reliably. Organic molecules having CF₃- as the terminal characteristic group are more easily oriented, raising the molecular density of organic compounds when they are aligned on the substrate while the monolayers are formed. Therefore, hydrophobic monolayers can be prepared more reliably.

In the present invention, the organic molecule having a structure represented by (i) is preferably at least one organic molecule selected from the group consisting of the compounds represented by the following General Formulae (20) to (29) and the derivatives thereof.

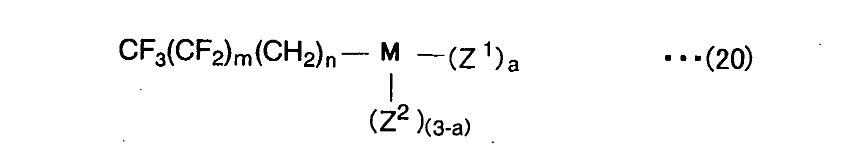

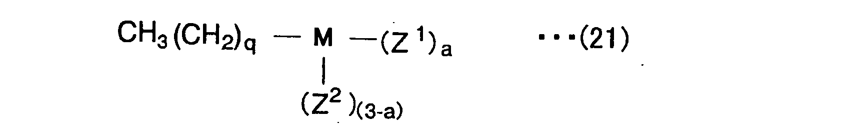

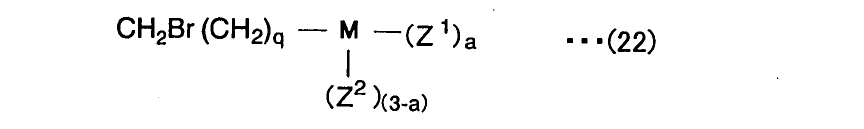

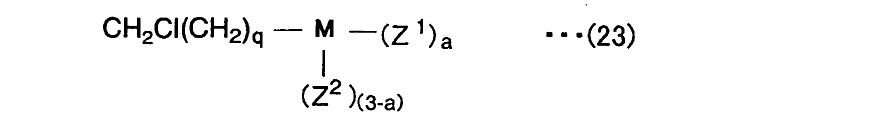

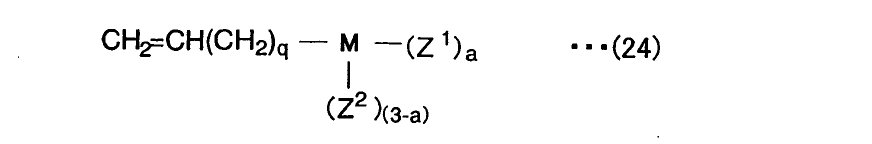

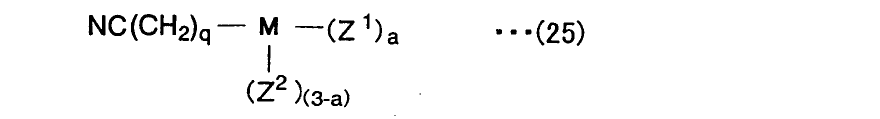

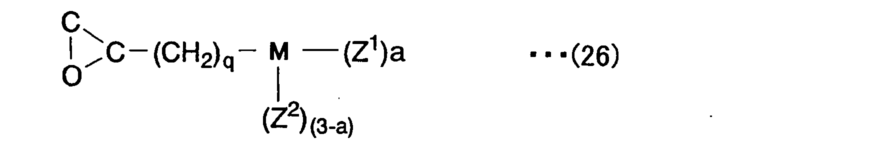

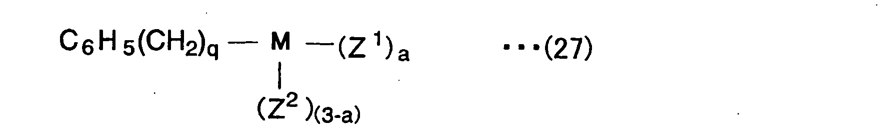

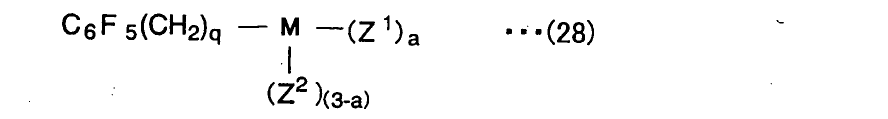

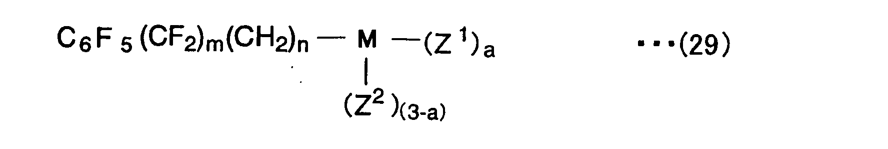

In General Formulae (20) to (29), M, Z¹, Z², and a are the same as those in General Formula (1) of organic molecule (i) above. q is an integer of 2 to 22. m and n each are an integer satisfying at the same time the conditions shown in the following Formulae (I) to (III): 0 ≤ m ≤ 14... (I); 0 ≤ n ≤ 15... (II); and 2 ≤ (m+n) ≤ 22... (III).

The organic molecule having a structure of (ii) is preferably at least one organic molecule selected from the group consisting of the compounds represented by the following General Formulae (30) to (39) and the derivatives thereof.

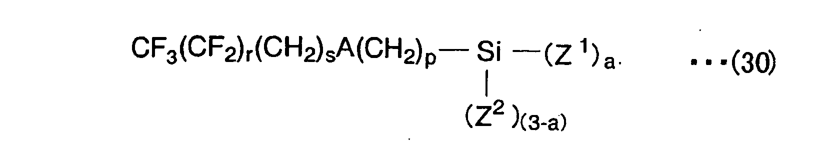

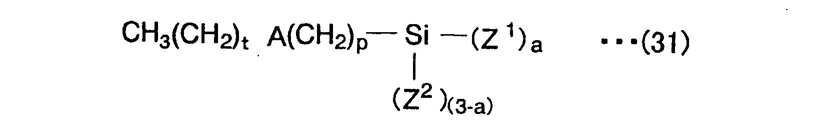

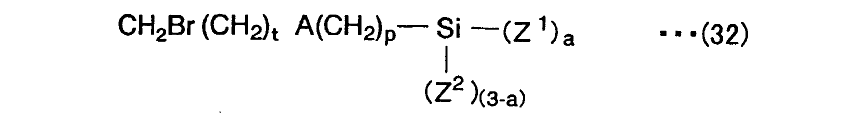

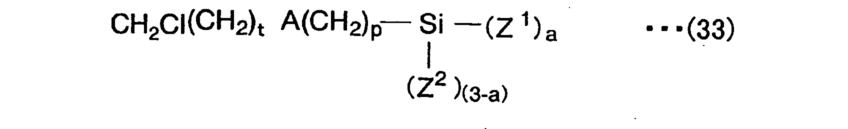

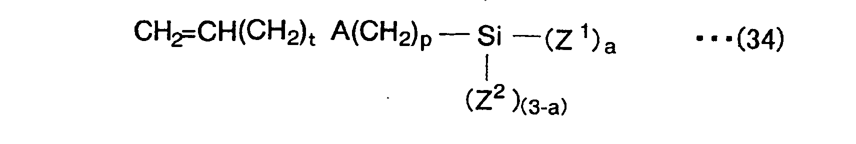

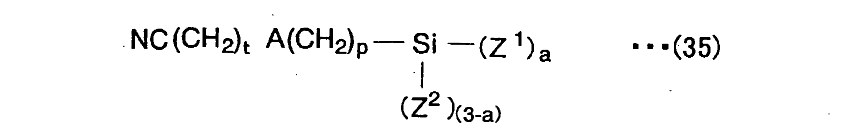

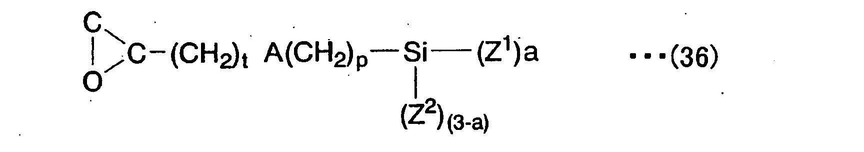

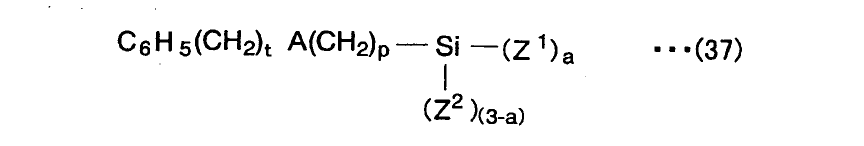

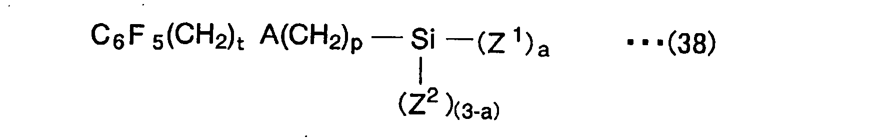

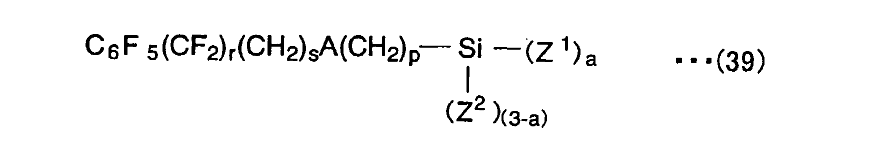

In General Formulae (30) to (39), Z¹, Z² and a are the same as those in General Formula (1) of organic molecule (i) above. A represents at least one bivalent characteristic group selected from the group consisting of the characteristic groups represented by General Formula (3), -O-, -COO-, -C₆H₄-and the derivatives thereof. t is an integer of 1 to 10. p is an integer of 1 to 18. r and s each are an integer satisfying at the same time the conditions shown by the following Formulae (IV) to (VI): 0 ≤ r ≤ 14... (IV); 0 ≤ s ≤ 15... (V); and 2 ≤ (r+s) ≤ 22... (VI).

The organic molecule having the structure of (iii) is preferably at least one organic molecule selected from the group consisting of the compounds represented by the following General Formulae (40) to (49) and the derivatives thereof.

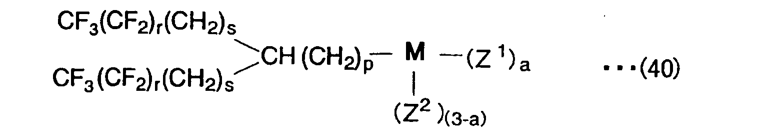

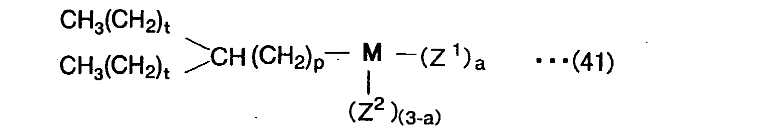

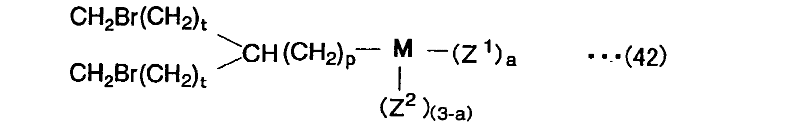

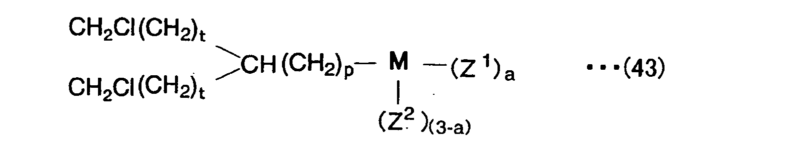

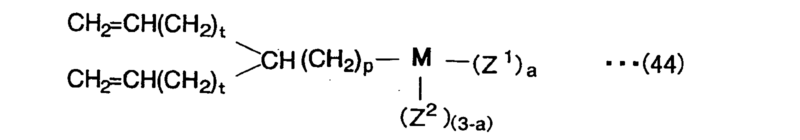

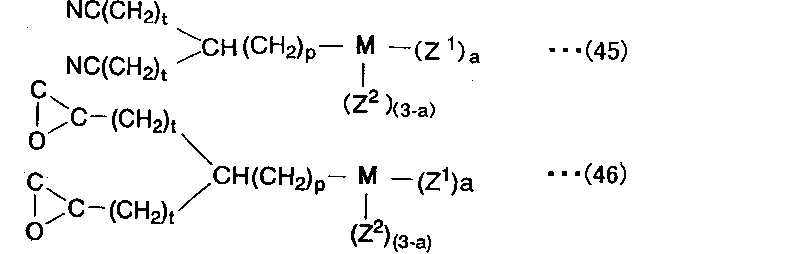

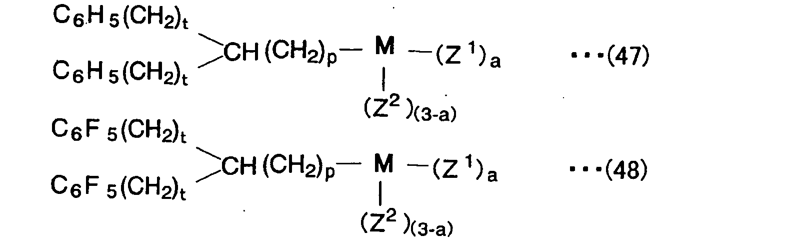

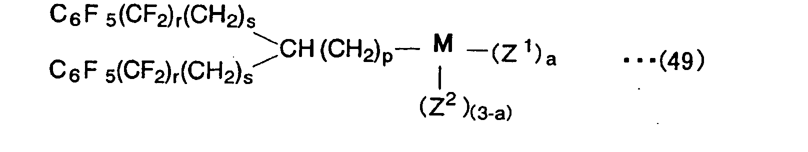

In General Formulae (40) to (49), M, Z¹, Z² and a are the same as those in General Formula (1) of organic molecule (i) above. t is an integer of 1 to 10. p is an integer of 1 to 18. r and s each are an integer satisfying at the same time the conditions shown by the following Formulae (IV) to (VI): 0 ≤ r ≤ 14... (IV); 0 ≤ s ≤ 15... (V); and 2 ≤ (r+s) ≤ 22... (VI).

Among the organic molecules represented by General Formulae (20) to (29), organic molecules represented by General Formulae (20) and (21) are preferable, from the viewpoints of ensuring the uniformity of monolayer and the molecular density of organic compounds aligned on the particular area when the monolayer is formed.

From the same viewpoint as above, among the organic molecules represented by General Formula (20), the organic molecules represented by the following Formulae (201) to (203) are preferable:

CF₃(CF₂)₇(CH₂)₂SiCl₃ (201)

CF₃(CF₂)₇(CH₂)₂Al(OCH₃)₃ (202)

CF₃(CF₂)₇(CH₂)₂TiCl(CH₃)₂ (203)

Further, from the same viewpoint as above, among the organic molecules represented by General Formula (21), the organic molecules represented by the following Formulae (211) to (214) are preferable:

CH₃(CH₂)₇(CH₂)₂SiCl₃ (211)

CH₃(CH₂)₇AlCl(OC₂H₅)₂ (212)

CH₃(CH₂)₇TiCl(C₃H₇)₂ (213)

CH₃(CH₂)₄SnCl(C₃H₇)₂ (214)

Further, among the organic molecules represented by General Formulae (30) to (39), organic molecules represented by General Formulae (30) and (31) are preferable, from the viewpoints of ensuring the uniformity of monolayer and the molecular density of organic compounds aligned on the particular area when the monolayer is formed.

From the same viewpoint as above, among the organic molecules represented by General Formula (30), the organic molecules represented by the following Formulae (301) to (306) are preferable:

CF₃(CF₂)₃(CH₂)₂O(CH₂)₁₅SiCl₃ (301)

CF₃COO(CH₂)₁₅SiCl₃ (302)

CF₃(CF₂)₃(CH₂)₂Si(CH₃)₂(CH₂)₉SiCl₃ (303)

CF₃(CF₂)₇Si(CH₃)₂(CH₂)₉SiCl₃ (304)

CF₃(CH₂)₂Si(CH₃)₂(CH₂)₁₅SiCl₃ (305)

CF₃CH₂O(CH₂)₁₅SiCl₃ (306)

From the same viewpoint as above, among the organic molecules represented by General Formula (31), the organic molecules represented by the following Formulae (307) to (312) are preferable:

CH₃(CH₂)₃(CH₂)₂O(CH₂)₁₅SiCl₃ (307)

CH₃COO(CH₂)₁₅SiCl₃ (308)

CH₃(CH₂)₃(CH₂)₂Si(CH₃)₂(CH₂)₉SiCl₃ (309)

CH₃(CH₂)₇Si(CH₃)₂(CH₂)₉SiCl₃ (310)

CH₃(CH₂)₂Si(CH₃)₂(CH₂)₁₅SiCl₃ (311)

CH₃CH₂O(CH₂)₁₅SiCl₃ (312)

Among the organic molecules represented by the Formulae above (201) to (203), (211) to (214), and (301) to (312), the organic molecule represented by Formula (201) is most preferable.

As organic molecules other than those described above, organic molecules described in Jpn. Unexamined Patent Publication Nos. 4-13267, 4-236466, 10-180179, and 4-359031 may be used in the range that provides the advantageous effects of the invention, the contents of which are hereby incorporated by reference.

Basically, commercially available reagents may be used as these compounds, but these compounds can be prepared easily by the following methods.

Commercially available reagents include, for example, decyltrichlorosilane manufactured by Sigma-Aldrich, Inc and the like.

Typical examples of the production methods include the methods described in Jpn. Unexamined Patent Publication Nos. 2-138286 and 4-120082, the contents of which are hereby incorporated by reference.

Specifically, organic silane compounds represented by General Formula (20) are obtained in the process of reacting a terminal perfluoroalkyl halogen compound represented by the following General Formula:

F(CF₂)_{α}(CH₂)_{β}X¹ (20a)

(in the Formula, α is an integer of 1 to 8; β is an integer of 0 to 2; and X¹ represents a halogen atom of I, Br or Cl); and a Grignard reagent

X²Mg(CH₂)_{γ}CH=CH₂ (20c)

prepared from a terminal alkenyl halide compound represented by the following General Formula:

X²(CH₂)_{γ}CH=CH₂ (20b)

(in the Formula, γ is an integer of 8 to 17; and X² is a halogen atom of I, Br or Cl)
to give a terminal perfluoroalkene compound represented by the following General Formula:

General Formula F(CF₂)_{α}(CH₂)_{β+γ}CH=CH₂ (20d);

and a process of reacting a terminal perfluoroalkene compound represented by General Formula (20d), and
a hydrogen silane represented by the following Formula:

HSi(CH₃)_{δ}X³ _{3-δ} (20e)

(in the Formula, δ is an integer of 0 to 2; and X³ is a halogen atom of I, Br or Cl or an alkoxy group) in a hydrosilylation reaction.

The hydrosilylation reaction is preferably carried out in the presence of a platinum catalyst.

In addition, the trifluoroalkylsilane compound of General Formula (21) is obtained in the process of hydrosilylating the ω-trifluoroalkene compound represented by the following General Formula:

CF₃(CH₂)_{ε}CH=CH₂ (21a)

(in the Formula, ε is an integer of 7 to 16);
with the aforementioned hydrogen silane represented by the following General Formula:

HSi(CH₃)_{δ}X³ _{3-δ} (21e).

The terminal perfluoroalkyl halogen compounds represented by General Formula (20a) are commercially available short chain compounds and include, for example, F(CF₂)₂CH₂Cl, F(CF₂)₂CH₂I, F(CF₂)₃I, and F(CF₂)₃CH₂Br.

The terminal alkenyl halogenated compounds represented by General Formula (20b) include, for example, Cl(CH₂)₁₀CH=CH₂, Cl(CH₂)₁₄CH=CH₂, and Br(CH₂)₁₇CH=CH₂. The hydrogen silanes represented by General Formula (20e) include, for example, HSiCl₃, HSi(CH₃)Cl₂, HSi(CH₃)₂Cl, HSi(OMe)₃, and HSiCH₃(OC₂H₅)₂.

The Grignard reagents represented by General Formula (20c) are prepared, for example, by first placing magnesium metal in a reaction solvent such as diethylether, tetrahydrofuran, or the like, and then supplying a terminal alkenyl halogenated compound of General Formula (20b) into the solution, for example, at 50 to 60°C. The amount of magnesium metal is preferably equimolar to or slightly higher than that of the terminal alkenyl halogenated compound.

The terminal perfluoroalkene compound of General Formula (20d) is prepared by reacting the Grignard reagent of General Formula (20c) prepared with a terminal perfluoroalkyl halogen compound of General Formula (20a) at room temperature in Grignard reaction. In a similar manner to above, the aforementioned Grignard reagent is gradually added into a reaction solvent such as diethylether, tetrahydrofuran, or the like containing the terminal perfluoroalkyl halogen compound of General Formula (20a). Alternatively, the terminal perfluoroalkyl halogen compound may be added into a reaction solvent containing the Grignard reagent. Cu may also be added as a catalyst. After completion of the reaction, water is added to the reaction system to dissolve the magnesium salt generated, and then the resulting organic and aqueous phases are separated. The terminal perfluoroalkene compound of General Formula (20d) is obtained after low-boiling compounds such as the reaction solvent and others are removed from the organic phase. If possible, the terminal perfluoroalkene compound may be purified by distillation.

The desired terminal perfluoroalkyl silane compound is obtained by reacting the terminal perfluoroalkene compound of General Formula (20d) and the hydrogen silane of General Formula (20e), for example, at about 100°C in hydrosilylation reaction.

In addition, the organic silane compound represented by General Formula (30) is typically prepared, for example, in the following process:

For example, the organic silane compound represented by General Formula (30) is prepared by reacting a relatively cheaper commercial compound represented by the following General Formula (30a):

L-C_{b}E_{2b}-Si(C_{g}H_{2g+1})(CₕH₂ₕ₊₁)-Z³ (30a)

[wherein, the terminal group L is at least one characteristic group selected from the group consisting of methyl group, halogen-substituted methyl groups, vinyl group, cyclic ether groups having 2 to 4 carbons, phenyl group, halogen-substituted phenyl groups, cyano group, and the derivatives thereof; C_{b}E_{2b} is the same as that in General Formula (2); C_{g}H_{2g+1} and CₕH₂ₕ₊₁ are the same as those in General Formula (3); and Z³ is Cl or OCH₃.], and the Grignard reagent represented by General Formula (30b):

CH₂=CH(CH₂)_{θ}X⁴ (30b)

[θ is an integer of 1 to 16; and X⁴ is a halogen.];
to give a compound represented by General Formula (30c):

L-C_{b}E_{2b}-Si(C_{g}H_{2g+1})(CₕH₂ₕ₊₁)-(CH₂)_{θ}CH=CH₂ (30c);

and further reacting the compound above in hydrosilylation reaction with a hydrogen silane represented by General Formula (30d):

HSi(Z¹)ₐ(Z²)₃₋ₐ (30d)

[Z¹, Z² and a are the same as those in General Formula (1).]

Silane compounds represented by General Formula (30a) include, for example, CF₃(CH₂)₂(CH₃)₂SiCl and CF₃(CH₂)₂(CH₃)₂SiOCH₃.

The hydrosilylation reaction may be carried out at a reaction temperature of 50 to 150°C in the presence of a catalyst, under reflux if the reaction is an atmospheric reaction, or in a state sealed in an autoclave if it is a pressurized reaction, by reacting the equimolar amounts of a terminal perfluoroalkene compound and a silicon compound, or by reacting the terminal perfluoroalkene compound with an excessive amount of the silicon compound as needed for completion of the reaction, as terminal perfluoroalkene compounds are generally expensive.

Further, an inactive hydrocarbon solvent such as n-hexane, isooctane, toluene, xylene, or the like may be used as needed in the reaction.

The product is sufficiently pure and usable when the low-boiling compounds including unreacted compounds, reaction solvents, and the like are stripped off after reaction, but may be further purified by distillation if the product is distillable.

Hereinafter, the methods of producing the device substrate according to the present invention will be described.

A substrate inherently having a characteristic group with active hydrogen may be used. Also, if the substrate does not have a sufficient amount of active hydrogens, a substrate of which the surface is provided before use with active hydrogens by surface treatment may be used. In particular, if a dense monolayer is desirably formed, the following surface treatment is preferable.

Methods of providing the substrate with active hydrogens include, for example, methods of oxidizing the surface chemically, treating with plasma in the presence of oxygen, and treating with ozone. The methods also include the method of hydrophilizing the substrate, for example, with SiCl₄, HSiCl₃, SiCl₃O-(SiCl₂-O)_{η}-SiCl₃ (wherein, η is an integer of 0 to 6), Si(OH)₄, HSi(OH)₃, Si(OH)₃O-(Si(OH)₂-O)_{η}-Si(OH)₃ (wherein, η is an integer of 0 to 6), or the like.

The method of oxidizing the surface will be described in more detail. The oxidation treatment may be performed, for example, by high-energy irradiation of the surface in the presence of oxygen and a hydrogen atom-supplying substance. For example, oxygen in air is decomposed by ultraviolet ray irradiation generating ozone, which in turn reacts with the hydrogen atom-supplying substance and generates active species having an active hydrogen. When the surface is irradiated with ultraviolet ray, covalent bonds of the atoms in the shallow surface of the material are cleaved, generating unbonded radicals.

Substrates having active hydrogens are obtained by the reaction of the unbonded radicals with active species containing active hydrogens. Water, ammonia, or the like, for example, is favorably used as the hydrogen atom-supplying substance, from the viewpoints of availability and convenience in handling. If water is used as the hydrogen atom-supplying substance, characteristic groups at least containing the structure represented by -OH are formed. Alternatively, if ammonia is used, characteristic groups at least containing the structure represented by -NH are formed. Corona discharge, oxygen plasma, or other treatment may be used, replacing the ultraviolet-ray irradiation treatment.

Subsequently, the monolayer non-compatible with liquid is formed on the substrate surface in flow channels in the monolayer-forming process, i.e., by preparing the substrate obtained as described above and coating a certain area therewith on the substrate surface in the flow channel.

If a device substrate is produced by the method of manufacture according to present invention, the non-compatible monolayer may be formed, for example, over the entire internal surface of the flow channel in substrate as described as an embodiment of the invention.

The methods of producing the monolayer are not particularly limited to the methods below, and include those disclosed, for example, in Jpn. Unexamined Patent Publication Nos. 4-13267, 4-236466, and 10-180179, and others, the contents of which are hereby incorporated by reference.

The monolayer can be formed by bringing the organic molecule above into contact with the substrates having active hydrogens in the manner described above. Although the contact treatment may be carried out either in a gas or liquid phase, liquid phase treatment is preferred because of simplicity in manufacture.

In the liquid phase treatment, the substrate is brought into contact with an organic molecule-containing solution, which is prepared by dissolving or suspending the organic molecule in a solvent. The solvent is preferably an aprotic solvent, and the moisture in the gas phase in contact with the organic molecule-containing solution is preferably controlled to a relative humidity of 35% or less, as expressed as a relative humidity at 22°C.

For ensuring production of a very thin monolayer uniform in thickness, moisture in the gas phase is preferably 25% or less, more preferably 5% or less, as expressed as a relative humidity at 22°C

Further, the reaction container for use in the process is preferably a sealed system such as a glove box or the like. The constituent gas of the gas phase of which the moisture is controlled in the range above is preferably at least one gas selected from the group consisting of noble gases and nitrogen gas. However, air may be used under a condition that sufficiently suppresses oxidation of the organic molecule or aprotic solvent and oxidative degradation of the monolayer. For example, air may be used by properly adjusting the temperature of the gas phase and the organic molecule-containing solution, the concentration of organic molecule, the contact time between the organic molecule and the substrate, and the like in the monolayer-forming process.

In a liquid phase treatment, an organic molecule-containing solution is first prepared by dissolving the organic molecule in a solvent. The aprotic solvent for use in preparing the organic molecule-containing solution may be selected arbitrarily according to the kind of the organic molecule, but is preferably a solvent that dissolves the organic molecule significantly, from the viewpoint of ensuring easy and reliable production of monolayers thinner in thickness (0.5 to 50 nm) and excellent in the uniformity of layer thickness. Examples thereof include organic solvents such as hexadecane, chloroform, carbon tetrachloride, silicone oil, hexane, toluene, and the like. These solvents may be used alone or in combination of two or more solvents.

Among them, mixed solvents containing hexadecane, chloroform and carbon tetrachloride are preferable as the aprotic solvent. Use of the organic solvent in this manner prevents sufficiently, for example, polymerization of the organic molecule caused by the presence of water. The use of the organic solvent facilitates the condensation reaction between the terminal bonding functional group of organic molecule and the active hydrogens of substrate.

The concentration of the organic molecule-containing solution is preferably 10⁻⁴ mol/L or more, more preferably 10⁻³ mol/L or more, and most preferably 10⁻¹ mol/L or less.

The contact time of the substrate with the organic molecule-containing solution is not particularly limited, but, for example, several seconds to 10 hours and preferably 1 minute to 1 hour. In addition, the temperature of the organic molecule-containing solution is, for example, 10 to 80°C and preferably in the range of 20 to 30°C.

In this manner, the organic molecules are bound to the substrate via covalent bonds [for example, siloxane bond (-Si-O-)], giving a monolayer.

The monolayer formed in this manner has characteristic groups non-compatible with the liquids described above on the surface, and thus the area on the device substrate according to the present invention where the monolayer is formed, i.e., the entire area of the flow channel, becomes non-compatible with the liquid.

Different from the embodiment above, a method of forming areas non-compatible with the liquid selectively only on parts of the flow channel will be described below.

The methods of forming the monolayer selectively on the substrate surface in the flow channel are not particularly limited and include, for example, the methods described below. The chemical absorbent solution, the processing condition, and the like used below are the same as those above, except otherwise specified.

The first method is a method of forming a monolayer over the entire substrate surface in flow channels and then removing only the monolayer formed in the area whereon the monolayer is not needed, i.e., the compatible area. After a monolayer is formed on the substrate surface, a photomask is additionally formed on the areas to be compatible with liquids of the monolayer (i.e., area wherein the monolayer is not needed). The monolayer on the area not covered by the photomask can be removed by irradiating with high-energy ray, for example, ultraviolet ray. When a laser such as an excimer laser or the like is used as the means for the ultraviolet ray irradiation, the ultraviolet ray may be spotted onto particular areas of the monolayer.

In addition to the ultraviolet-ray irradiation treatment, other method such as corona, plasma, or other treatment may be employed. By conducting these treatments in the presence of oxygen, the monolayer on the substrate surface may be removed by oxidation.

In addition, it is possible to remove the monolayer directly without use of the photomask by using a pulsed laser. The basic principle of the removal of organic films by using pulsed laser is described in R. Srinivasan and W. J. Leigh, J. Amer. Chem. Soc., 104, 6784 (1982), the contents of which are hereby incorporated by reference.

In the second method, a monolayer is selectively formed on a substrate surface by forming a resist pattern that covers only the compatible area in the substrate surface in flow channels, bringing the organic molecule described above into contact with the substrate surface whereon the resist pattern is formed, and removing the resist pattern.

First, a resist pattern for covering the compatible area is formed. The resist pattern can be formed easily by a semiconductor film-producing technology. Subsequently, a monolayer is selectively coated on the area not covered by the resist, by bringing an organic molecule to the substrate whereon the resist pattern is formed, and then the resist pattern is removed, to form non-compatible areas. The resist pattern may be a positive or negative resist pattern.

As the method of bringing the organic molecule into contact with the substrate in this process, preferably is the following method. Namely, first, an organic molecule-containing solution is prepared by dissolving the organic molecule in an aprotic solvent. Then, the organic molecule-containing solution and the substrate after the resist pattern is formed are placed in a container such as a glove box or the like, of which the internal moisture in gas phase can be easily controlled in the range described above, to allow a condensation reaction to progress.

The aprotic solvent used for preparation of the organic molecule-containing solution may be arbitrarily selected according to the kind of the organic molecule, if it is a solvent that does not dissolve the resist pattern, but is preferably a fluorine-based solvent, from the viewpoint of obtaining a monolayer thinner in thickness (0.5 to 50 nm) and superior in the uniformity of layer thickness more easily and reliably. The preferable fluorine-based solvents include perfluorocarbon- and hydrofluoroether-derived liquids manufactured by Sumitomo 3M Limited. Specifically, the solvent is preferably "HFE-7200", "PF-5080", or "FC-77" (trade name, manufactured by Sumitomo 3M Limited), from the viewpoints of various physical properties of the solvent such as the boiling point suitable for the temperature condition of the processing. Reaction conditions such as the concentration and the reaction time of the organic molecule may be similar to those described above.

Subsequently, after the monolayer is formed, the resist pattern can be removed by using, for example, acetone.

In the third method, a monolayer is formed selectively by bringing an organic molecule into contact only with the non-compatible area by ink-jet, stamping, or other method. The monolayer may be a single layer or a laminated layer having multiple layers. For example, after a monolayer is formed on a substrate as described above, the second monolayer may be formed thereon. In such a case, if there are no reactive functional groups on the surface of the first monolayer, active hydrogens may be provided by the surface treatment described above.

More specifically, if a terminal group not binding to a covalent bond of the first monolayer is a characteristic group containing a group having an unsaturated bond such as a vinyl group or the like, a characteristic group having at least the structure of -OH can be introduced by irradiating an energy beam such as electron beam, X ray, or the like onto the surface of monolayer under an environment containing moisture and thus changing part of the unsaturated bond-containing group. If a characteristic group having a an unsaturated bond-containing group such as a vinyl group or the like is bound to the substrate, a characteristic group having at least the structure of -COOH can be introduced, for example, by immersing the substrate in an aqueous potassium permanganate solution and thus changing the structure of part of the unsaturated bond-containing group.

The thickness of the monolayer may be arbitrarily selected according to the kind (length) of organic molecule or by forming the laminated layer above; or adjusted, for example, by the method of binding additionally a non-compatible molecule to the terminal of the terminal characteristic group of the organic molecule constituting the monolayer after forming the first monolayer.

If the organic molecule constituting the monolayer has a double bond or triple bond at the terminal of the terminal characteristic group as shown in General Formula (24), the monolayer may be additionally brought into contact, for example, with a Grignard reagent (RMgX) after it is formed. The contact results in an addition reaction between the terminal characteristic group and RMgX, resulting in connection of the hydrocarbon group (R-) in RMgX to the terminal of the terminal characteristic group. In RMgX, R is an alkyl group, halogenated alkyl group, alkenyl group, or halogenated alkenyl group having 1 to 23 carbons; and X is a halogen (F, Cl, Br, or I).

Alternatively, if the organic molecule constituting the monolayer has an epoxy group as the terminal characteristic group as shown in General Formula (26), the monolayer may be additionally brought into contact with an alcohol (ROH) after it is formed. The contact allows progress of the ring-opening (addition) reaction of epoxy groups, resulting in connection of the "R group" in alcohol (ROH) to the third characteristic group. In ROH, R is an alkyl group, halogenated alkyl group, alkenyl group, or halogenated alkenyl group having 1 to 23 carbons; and X is a halogen (F, Cl, Br, or I).

A desirable device substrate is obtained after the monolayer is formed in the manner described above. If the liquid is an aqueous solution and the hydrophilicity is not satisfactory or desirably increased, the hydrophilicity may be enhanced, for example, by repeating the surface treatment selectively on the areas in flow channels.

In another preferred embodiment of the present invention, a hydrophilic monolayer is formed on the interior of the flow channels for the hydrophilic surface treatment.

Namely, in a similar manner to the aforementioned hydrophobic monolayer-forming process, a hydrophilic monolayer may be formed inside the flow channel on the substrate. Favorably, the hydrophilic monolayer further increases the difference in hydrophilicity between the hydrophilic areas and the hydrophobic monolayer areas formed surrounding them.

In this embodiment, (A) an organic molecule having a terminal functional group capable of forming a covalent bond with a substrate and other terminal group hydrophile with a liquid, or (B) an organic molecule having a terminal functional group capable of forming a covalent bond with a substrate and other terminal group which can be modified to a hydrophilic group after the monolayer may be used.

Examples of the organic molecules (A) in the former group, which have a hydrophilic terminal group, include organic molecules represented by the following General Formula (50).

T¹-(C_{λ}H_{2λ})-Si-(Z⁴)_{ϕ}(Z⁵)_{3-ϕ} (50)

[T¹ represents a CHO group, COOH group, OH group, NH₂ group, COOR group, PO(OH)₂ group, PO(OH) group, SO₃H group, SO₂H group or SH group; λ is a number required for making the total molecular length of the compound including the terminal functional group above shorter than or as small as the molecular length of the organic group that forms hydrophobic monolayer; Z⁴ represents an alkoxy groups having 1 to 5 carbons; Z⁵ represents at least one atom or atomic group selected from the group consisting of H and alkyl groups having 1 to 5 carbons; and ϕ is an integer of 1 to 3.]

The organic molecules include those wherein the terminal group not forming a covalent bond with the substrate surface is substituted with a group having an active hydrogen such as a hydroxyl, amino, or other group. These organic molecules are immobilized tightly on the substrate by forming a covalent bond when they are brought into contact with the substrate. Because the terminal group opposite to the covalent bond is a hydrophilic group, the liquids flowing in flow channels become in contact only with the areas where the hydrophilic monolayer is formed.

In addition, spreading of the droplets is suppressed, as the surrounding area is coated with a hydrophobic monolayer.

Methods similar to the aforementioned hydrophobic monolayer-forming processes may be used as the hydrophilic monolayer-forming process above.

Organic molecules having a hydrophilic terminal group include the following compounds (501) to (508):

H₂N(CH₂)₃Si(OCH₃)₃ (501)

OHC(CH₂)₃Si(OCH₂CH₃)₃ (502)

HOOC(CH₂)₅Si(OCH₃)₃ (503)

HO(CH₂)₅Si(OCH₃)₃ (504)

H₃COOC(CH₂)₅Si(OCH₂CH₃)₃ (505)

(OH)₂OP(CH₂)₃Si(OCH₃)₃ (506)

HO₂S(CH₂)₃Si(OCH₃)₃ (507)

HS(CH₂)₃Si(OCH₃)₃ (508)

The latter organic molecules (B) of providing a monolayer withhydrophilicity is a method of substituting the terminal group of a compound that is hydrophobic when prepared with a hydrophilic group by the aforementioned monolayer-forming process. For example, a hydroxyl group (-OH) may be introduced to an organic molecule having a double bond at the terminal, by irradiating the molecule with a high-energy beam such as electron beam, X ray, or the like under an environment containing moisture.

It is also possible to introduce a carbonyl group (-COOH), for example, by immersing the substrate in an aqueous potassium permanganate solution. If the compound has, for example, a cyano group (-CN group), the cyano group may be converted to an amino group by reducing it in a lithium aluminum hydride solution. Alternatively, the ring-opening reaction of an epoxy group may also be used.

Such organic molecules include those represented by the following General Formula (60):

T²-(CᵥH₂ᵥ)-Si-(Z⁶)_{ρ}(Z⁷)_{3-ρ} (60)

[wherein, T² represents a vinyl, methyl, halogenated methyl, epoxy, or cyano group; v is a number required for making the total molecular length of the compound including the terminal functional group above shorter than or as short as the molecular length of the organic group that forms hydrophobic monolayer; Z⁶ represents at least one atom or atomic group selected from the group consisting of F, Cl, Br, I, -OH, -SCN, -NCO, and alkoxy groups having 1 to 5 carbons; Z⁷ represents at least one atom or atomic group selected from the group consisting of H and alkyl groups having 1 to 5 carbons; and p is an integer of 1 to 3.]

Covalent bonds are formed and thus a monolayer is formed by bringing the organic molecule having the terminal group above into contact with the substrate. For example, if the terminal group is a double bond, the terminal double bond can be converted into a hydrophilic carboxyl group by bringing the compound into contact with an aqueous potassium permanganate solution.

The organic compounds capable of providing a hydrophilic group at the terminal include the following organic molecules (601) to (603):

CH₂=CH(CH₂)₆Si(OCH₃)₃ (601)

CH₃C₆H₄Si(OCH₃)₃ (602)

ClCH₂C₆H₄Si(OCH₃)₃ (603)

Preferably, the total carbon number of the characteristic group of the organic compound above present between the terminal bonding functional group capable of forming a covalent bond and the hydrophilic terminal group is properly determined so that the molecular length of the organic molecule becomes shorter than that of the non-compatible monolayer surrounding the area where the organic molecule is coated as a monolayer.

If a hydrophilic monolayer described above is formed, the degree of hydrophilicity is determined relative to the surrounding hydrophobic monolayer, and thus the hydrophilic monolayer should only have a greater critical surface energy than that of the hydrophobic monolayer. For example, if liquid containing water is used, the critical surface energy thereof is preferably more than 20 mN/m, more preferably 60 mN/m or more, and more preferably 75 mN/m or less.

If a surface treatment is to be carried out by using the organic molecule described above, the hydrophilic monolayer may be formed after a hydrophobic monolayer is formed or in advance to formation of a hydrophobic monolayer.

Accordingly, since a monolayer of the present invention may be changed the compatibility with a liquid, the particular area of the flow channel can be coated with a monolayer non-compatible with the liquid, i.e., with the monolayer above according to the present invention, properly according to the liquid to be placed. Therefore, even if the liquid to be placed is not an aqueous solution, the present invention may be used in an analogous manner for confining the liquid in particular areas by using the difference in affinity to the liquid.

Hereinafter, the structure of the integrated microchip employing the device substrate according to the present invention will be described. These structures are provided only as examples, and the device substance according to the invention may be used as the device substrates for use in other microchips as well.

First, the substrates made only of the device substrate according to the present invention may be applied to microchips. Substrates in the structure having compatible areas in flow channels may be used in such applications.

In addition, if analysis is desirably carried out under an environment where the substrate is less exposed to external atmosphere, the substrate according to the present invention may be in the structure wherein it is adhered to another substrate.

For example, a microchip is prepared by using the device substrate having flow channels according to the present invention as the bottom substrate and conjugating a top substrate thereto. A substrate having a non-compatible monolayer is favorable used as the top substrate. Alternatively, another substrate may be placed atop the substrate via a spacer or the like, in the manner that it faces the substrate described above. In such a case, it is preferable that the other substrate atop preferably has through-holes, for example, for addition of samples, reagents, and the like, and for degasification.

The bottom substrate has reagent-storing, reaction, measurement, and other units, and flow channels are connected to respective units. Substrate, enzyme, reaction-terminating, and other solutions are injected through the through-holes previously placed in the top substrate into the respective units. The internal surface of respective storing units is preferably coated with a monolayer non-compatible with the liquids to be placed as well.

The transportation of respective solutions and the reaction solutions are controlled by a micropump or the like, and the reagents fed into a predetermined reaction unit react therein. The reaction time in this reaction unit is properly determined and the reaction is discontinued by injection of a reaction-terminating solution after a particular period of time. The reaction solution is fed via a flow channel into a measurement unit for measurement by a spectroscopic method or the like.

In the configuration above, the device substrate according to the present invention may be used for the method of analyzing samples that requires adjustment of the flow rate of liquids by modifying the monolayer, and the flow rate of the liquids may be changed by properly selecting the area and shape of the monolayer in the internal surface of flow channels and the kind of the monolayer. Further, if the monolayer coating the internal surface of flow channels is formed by two or more kinds of monolayers different in compatibility with the liquids, it becomes possible to change the flow rate of the liquids by modifying the difference in compatibility. In yet another embodiment of the invention, the microchip above may be used in microchip modules in combination with the detection means known in the art.

Hereinafter, the present invention will be described in more detail with reference to EXAMPLES, but the structure, production process, and material according to the present invention may be modified arbitrarily and it should be understood that the present invention is not limited to the structure, production process, and material set forth above and below. In addition, the terms here are only used for the purpose of describing specific embodiments and should not be considered to be restrictive, as the scope of the invention is restricted only by the claims attached and the equivalents thereof.

### EXAMPLE

### [Production of device substrates and microchips]

### EXAMPLE 1

Microchips are produced as follows:

First, a glass substrate (length: 76 mm, width: 26 mm, and thickness: 2 mm) having units for storing a substrate solution, enzyme solution, and reaction-terminating solution, reaction unit, and measurement unit was prepared as a bottom substrate. Flow channels are connected to respective units (substrate-storing unit, enzyme-storing unit, reaction-terminating solution-storing unit); a reaction unit is placed at the intersection of the flow channels; and another flow channel is additionally connected to the reaction unit. The flow channel connected to the reaction unit is further connected at the other end to a measurement unit, to which yet another flow channel is connected, which in turn is connected to a discharge outlet. The outlet is connected to an external tube for discharge. The flow channels above are in the shape of groove having a width of 100 µm and a depth of 30 µm, and the respective storing units and the reaction unit are columnar dents having a diameter of 3 mm and a depth of 30 µm.

The glass substrate was washed with pure water and air-dried, then washed with ethanol and air-dried, and washed with ultraviolet-ray and ozone. The resulting glass substrate was stored in a glove box to which nitrogen gas having a dew point of -55°C is supplied.

The glass substrate was brought into contact with the trichlorosilane compound described above, as the glass substrate was immersed in a 1.0 weight % non-aqueous solution containing F(CF₂)₈(CH₂)₂SiCl₃ (hydrofluoroether; trade name: HFE7100; manufactured by Sumitomo 3M Limited) at room temperature.

Then, after removal of the trichlorosilane compound physically adsorbed on the glass substrate by washing, a monolayer was formed via covalent bonds on the surface of the glass substrate having the grooves (flow channels) and the dents described above.

Subsequently, a glass substrate (length: 76 mm, width: 26 mm, and thickness: 1 mm) was made available as the cover (top substrate) of the bottom substrate, and a monolayer was formed thereon in a similar manner to the bottom substrate. The resulting top substrate was adhered to the bottom substrate with the monolayer-formed surface facing the bottom substrate, to give a microchip.

### EXAMPLE 2.

A substrate (length: 76 mm, width: 26 mm, and thickness: 2mm) whereon two grooves are formed crosswise was made available, and a monolayer was formed on the substrate surface (including the internal surface of grooves) under a condition similar to that of the EXAMPLE 1. The width and the depth of the grooves were both 120 µm, and the distances between the intersection of the cross and the ends of the grooves were 10 mm. A top substrate having a monolayer similar to that of EXAMPLE 1 was placed on the substrate, to give a microchip.

### EXAMPLE 3.

A glass substrate having units for storing substrate solution, enzyme solution and reaction-terminating solution, a reaction unit, and a measurement unit (length 100 mm, width: 35 mm, and thickness: 2mm) was prepared in the similar manner to EXAMPLE 1 as a bottom substrate. Used was a substrate whereon flow channels (width 100 µm, depth 30 µm) were connected to respective storing units (substrate-storing unit, enzyme-storing unit, reaction-terminating solution-storing unit).

Separately, a mixed solution of n-hexane and chloroform (volume ratio 4:1) containing CH₃(CH₂)₁₇SiCl₃ at a concentration 1.0 weight % was prepared.

A monolayer was formed in a similar manner to EXAMPLE 1, except that the glass substrate and the organic molecule-containing solution above were used.

Subsequently, a photomask was placed on the glass substrate whereon the monolayer was formed, for forming hydrophilic areas on the flow-channel bottom faces. Ultraviolet ray (light source: 110-W lamp, manufactured by SEN light Corporation) was irradiated for 8 seconds through the photomask. It was found that a device substrate wherein the substrate is exposed in the flow channels could be produced by adjusting the irradiation width at 1 µm.

The fact that the monolayer in the areas irradiated with ultraviolet ray was removed was confirmed by monitoring the presence or absence of absorption corresponding to the CH₂ bond in the infrared absorption spectrum and the wettability of the areas to water.

### EXAMPLE 4.

The flow channels of the glass substrate used in EXAMPLE 3 was coated with a positive resist; the photomask identical with that used in EXAMPLE 3 was placed on the substrate; and a resist pattern was formed thereon via a resist-exposure process, developing process, and cleaning process.

Subsequently, a monolayer was formed in the similar manner to EXAMPLE 1 and then the resist pattern was removed by using a developing solution. It is confirmed that it is possible to prepare a device substrate having some areas in the flow channels wherein the substrate was exposed.

### EXAMPLE 5.

A hydrophobic monolayer was formed on the entire surface in the flow channels of the substrate in the analogous manner to the preparation of the monolayer of EXAMPLE 3.

The substrate was irradiated with a pulsed laser (540 nm, femtosecond pulse, output: 13 mW, and laser beam diameter: 1.5 µm). It was confirmed that it was possible to prepare by laser irradiation a device substrate having hydrophilic areas on the bottom faces of flow channels along the traveling direction of liquids.

### EXAMPLE 6.

The device substrate prepared in EXAMPLE 5 was hydrophilized as follows.

An aqueous alcohol solution containing H₂N(CH₂)₃Si(OCH₃)₃ (concentration: 0.5 weight %, and adjusted at pH 10) was coated on the device substrate prepared in EXAMPLE 5.

A solution was pushed away on the monolayer of the substrate and adhered only on the areas where the monolayer above was removed. After the solution was evaporated, the device substrate was treated in hot air at 120°C. By this treatment, a hydrophilic monolayer was coated on the areas on the glass substrate where the monolayer was removed. The areas became hydrophilic by the amino groups provided. The fact that a desired hydrophilic monolayer was formed was confirmed by monitoring the presence or absence of the absorptions corresponding to the CH₂ and NH₂ bonds in the infrared absorption spectrum.

### EXAMPLE 7.

The device substrate of EXAMPLE 3 whereon a compatible area was formed on the bottom faces of flow channels was used.

Part of the monolayer on the substrate was removed, for forming hydrophilic areas along the traveling direction of the liquids on the internal walls of flow channels on the glass substrate whereon the monolayer was previously formed. The monolayer was removed under the same condition as that of EXAMPLE 5. It was confirmed that it was possible to form hydrophilic areas on the side walls of flow channels along the traveling direction of liquid by laser irradiation and to prepare a device substrate having hydrophilic areas on the respective faces of the flow channels together with the hydrophilic areas on the bottom face.

Subsequently, for evaluation of properties, the microchips prepared in EXAMPLES 1 and 2 were subjected to the following evaluation tests, respectively together with the microchips for comparison whereon monolayer is not formed.

### [Evaluation tests]

### Experimental example 1

The microchips prepared in EXAMPLE 1 and a microchip whereon no monolayer is formed were evaluated as follows.

A substrate solution, an enzyme solution, and a reaction-terminating solution were injected into the respective storing units on each microchip through the through-holes which were previously placed in the top substrate in a similar manner to above. Transfer of the respective solutions and the reaction solution was controlled by a micorpump in a conventional manner.

### (Buffer solution)

A solution of 3.25 g of disodium hydrogen phosphate hydrate dissolved in 100 mL of water (pH 4.5) and a solution of 1.15 g of citric acid dissolved in 100 mL of water (pH: 4.5) were mixed at a ratio of 1:1, to give a buffer solution (pH: 4.5).

### (Enzyme solution)

β-Galactosidase was dissolved in the buffer solution, to give enzyme solutions at concentrations of 0.1 and 0.5 mg/mL.

### (Substrate solution)

Ortho-nitrophenylgalactosidase was dissolved in the buffer solution, to prepare a 0.1 mg/ml substrate solution.

First, the substrate solution and the enzyme solution were fed into the reaction unit to start the reaction. Ortho-nitrophenol was generated by the reaction of the enzyme and the substrate in the reaction unit. After certain periods of reaction time in the reaction unit (1, 2, 3, 5, and 10 minutes), the reaction-terminating solution was injected from the reaction-terminating solution-storing unit via the flow channel connected to the reaction unit, to discontinue the reaction. Then, the reaction solution after discontinuation was transferred via the flow channel leading to the measurement unit, wherein the absorption intensity thereof at a wavelength of 420 nm was determined by using ultraviolet and infrared spectrometer. The absorption wavelength of the ortho-nitrophenol is 420 nm.

Separately, the absorbance of the microchip for comparison prepared in a similar manner to EXAMPLE 1 except that both the bottom and top substrates were not coated with the monolayer was determined. As a result, the microchip of EXAMPLE 1 had an average of the absorbencies in multiple measurements (5 times) after respective reaction times of 0.98 and an error of 0.02 or less, when 0.5 mg/mL enzyme solution was used.

When 0.1 mg/mL enzyme solution was used, the average absorbance gradually increased over time, and the absorbencies thereof were 0, 0, 0.10, and 0.22 after respective reaction times. On the other hand the microchip for comparison had an average of the absorbencies in multiple measurements (5 times) after respective reaction times of 0.67 and an error of about 0.1, when 0.5 mg/mL enzyme solution was used. As apparent from the results above, the microchip of EXAMPLE 1 whereon a monolayer is formed is higher in measurement sensitivity and accuracy than that of COMPARATIVE EXAMPLE 1, even when the same enzyme at the same concentration was used. It is probably because transfer and mixing of the liquids are faster and the adhesion of the enzyme and substrate onto the flow channels are prevented.

### Experimental example 2

The microchips prepared from the substrate of EXAMPLE 2 and the substrate of EXAMPLE 2 having no monolayer formed were evaluated as follows.

A mixed DNA sample containing various DNA fragments was separated by electrophoresis, by injecting the DNA sample at one end of a groove and applying a voltage (100 V/cm) between one end and the other end of the groove. As a result, DNA separation from the mixed DNA solution was confirmed both on the microchips of EXAMPLE 2 and for comparison. However, examination of the separation accuracy by a spectroscopic method revealed that the accuracy of the microchip of EXAMPLE 2 is approximately 4 times higher than that of the microchip for comparison. The result indicates that it is possible to improve the separation accuracy and shorten the processing time only by forming a monolayer, for example, without elongation of the processing time, increase in the number of separation operations, or the like which was considered hitherto necessary.

In this manner, the device substrate according to the present invention, which has the non-compatible monolayer on the internal surface of flow channels permits faster transfer of the liquids in the flow channels, due to the change in wettability to the liquid caused by the noncompatibility of the monolayer.

In other words, presence of the non-compatible areas on the internal surface of the flow channels, for example, prevents adhesion and retention of the liquids on the internal surface caused by the surface tension or the like, enabling faster liquid transfer. In particular, the prevention of the retention of liquids described above permits effective use of trace amounts of samples.

It also allows uniform mixing of samples, reagents, and the like, as they are transferred in the flow channels. It is probably because, although there was turbulence in the flow of liquids through flow channels in the conventional microchips and the like, the turbulent flow can be changed to a laminar flow in this configuration of microchips.

Further, the layer on the substrate surface in flow channels is a monolayer having a thickness at the nano order, which cause no problems in visual observation and optical analysis. Further, the substrate and the monolayer are bound to each other via covalent bonds, extremely strong bonds, eliminating the concern about the separation of the monolayer during handling.

It is also possible to form covalent bonds between the substrate and the monolayer by bringing the aforementioned organic molecule into contact with the substrate having active hydrogens by the method of manufacture according to the present invention.

Therefore, it is possible to form minute non-compatible areas selectively without tedious operations.

In addition, use of the device substrate according to the present invention allows, for example, miniaturization of circuits and more efficient use of smaller amounts of samples, and thus the device substrate according to the present invention is useful in various areas including medical care, development of new drugs, analysis, and the like as the substrates for the various microchips described above, in particular for integrated microchips.

A device substrate comprising a substrate having a flow channel of a liquid, wherein an internal surface of said flow channel has an area non-compatible with said liquid; said area non-compatible with the liquid is formed by a monolayer non-compatible with said liquid; and said monolayer is connected to said substrate via a covalent bond, which allows smooth flow of the liquid in the minute flow channel.

## Claims

1. A device substrate comprising a substrate having a flow channel of a liquid, wherein:
an internal surface of said flow channel has an area non-compatible with said liquid;
said area non-compatible with the liquid is formed by a monolayer non-compatible with said liquid; and
said monolayer is connected to said substrate via a covalent bond.

2. The device substrate according to claim 1, wherein the entire internal surface of said flow channel is the area non-compatible with said liquid.

3. The device substrate according to claim 1, wherein the internal surface of the flow channel of said substrate has additionally an area compatible with said liquid; and the area non-compatible with said liquid and the area compatible with said liquid are formed along the traveling direction of the liquid.

4. The device substrate according to claim 3, wherein the area compatible with said liquid is an exposed area of the substrate.

5. The device substrate according to claims 3 or 4, wherein said area compatible with the liquid is formed continuously along said flow channel from one end to the other in the traveling direction of said liquid.

6. The device substrate according to any one of claims 3 to 5, wherein two or more of said areas compatible with the liquid are formed on the internal surface of said flow channel in the traveling direction of the liquid.

7. The device substrate according to any one of claims 3 to 6, wherein said internal surface of the flow channel has at least a bottom face and two side faces; and said areas compatible with the liquid are formed on said bottom face and two side faces respectively.

8. The device substrate according to any one of claims 1 to 7, wherein the covalent bond formed between said monolayer and said substrate is at least one bond selected from the group consisting of M-O, M-N and M-S bonds (M represents Si, Ti, Al or Sn).

9. The device substrate according to any one of claims 1 to 8, wherein:
said monolayer is connected to said substrate by forming at least one covalent bond selected from the group consisting of M-O, M-N and M-S bonds (M represents Si, Ti, Al or Sn) between said monolayer and said substrate; and
said monolayer comprises an organic group having
(i) a terminal group not bound to the substrate that is at least one characteristic group selected from the group consisting of methyl group, halogen-substituted methyl group, vinyl group, cyclic ether group having 2 to 4 carbons, phenyl group, halogen-substituted phenyl group, cyano group and the derivatives thereof and
(ii) a bivalent characteristic group represented by General Formula (S1) between said covalent bond and the terminal.
-C_{b}E_{2b}- (S1)
[ in the General Formula (S1), E represents at least one atom selected from the group consisting of H and F; and b is an integer of 2 to 22.]

10. The device substrate according to claim 9, wherein:
said monolayer is connected to said substrate by forming at least one covalent bond selected from the group consisting of Si-O, Si-N and Si-S bonds between said monolayer and said substrate; and
said monolayer comprises an organic group having additionally at least one bivalent characteristic group selected from the group consisting of characteristic groups represented by the following General Formula (S2), -O-, -COO-, -C₆H₄- and the substituted derivatives thereof between carbons constituting a carbon backbone of the bivalent characteristic group represented by General Formula (S1). 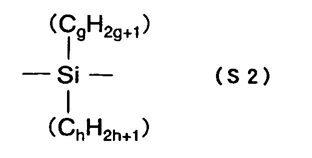 [In General Formula (S2), g and h each are independently an integer of 1 to 3.]

11. The device substrate according to any one of claims 1 to 8, wherein:
said monolayer is connected to the substrate by forming at least one covalent bond selected from the group consisting of M-O, M-N and M-S bonds (M represents Si, Ti, Al or Sn) between said monolayer and said substrate; and
said monolayer comprises an organic group having
(i) a terminal group not bound to said substrate that is at least one characteristic group selected from the group consisting of methyl group, halogen-substituted methyl group, vinyl group, cyclic ether group having 2 to 4 carbons, phenyl group, halogen-substituted phenyl group, cyano group and the derivative thereof and
(ii) a trivalent characteristic group represented by the following General Formula (S3) between the covalent bond and the terminal group not bound to said substrate. 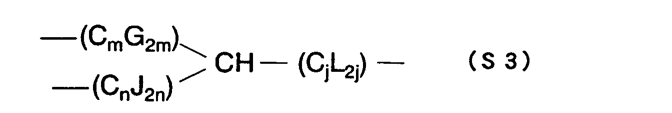 [in General Formula (S3), CⱼL₂ⱼ represents a characteristic group binding to the atom represented by said M; CₘG₂ₘ and CₙJ₂ₙ represent characteristic groups binding to the terminal group not binding to said substrate; G, J and L each represents independently at least one atom selected from the group consisting of H and F; j is an integer of 1 to 18; and m and n each are independently an integer of 0 to 7.]

12. The device substrate according to any one of claims 9 to 11, wherein the carbon number in a main chain of said bivalent characteristic group of General Formula (S1) or said trivalent characteristic group of General Formula (S3) is 8 or more and 18 or less.

13. A device substrate having a flow channel of a liquid, wherein:
an internal surface of said flow channel has an area non-compatible with said liquid;
said area non-compatible with the liquid is formed by a monolayer non-compatible with said liquid;
said monolayer is connected to said substrate by bringing an organic molecule into contact with said substrate and forming the covalent bond; and
said organic molecule has a first terminal group capable of forming a covalent bond with an active hydrogen on said substrate surface and a second terminal group non-compatible with the liquid.

14. The device substrate according to any one of claims 1 to 13, wherein an exposed area of the substrate whereon said monolayer non-compatible with the liquid is not formed on the internal surface of said flow channel has an active hydrogen.

15. A method of manufacturing a device substrate having a flow channel of a liquid formed thereon, comprises a step of forming an area non-compatible with the liquid by:
providing a substrate having an active hydrogen on a surface of said substrate in the flow channel;
preparing an organic molecule containing a bonding functional group capable of forming a covalent bond with the active hydrogen on said substrate surface at a terminal and a terminal group non-compatible with said liquid at the other terminal;
contacting said organic molecule with the substrate surface having the active hydrogen on said substrate; and
connecting a monolayer non-compatible with the liquid to said substrate surface by forming a covalent bond in a reaction of said terminal bonding functional group of the organic molecule and said active hydrogen of the substrate.

16. The method of manufacturing a device substrate according to claim 15, wherein the monolayer non-compatible with the liquid covers on a part of the substrate surface of the flow channel by forming said monolayer non-compatible with said liquid selectively on the substrate surface.

17. The method of manufacturing a device substrate according to claim 16, wherein the monolayer non-compatible with the liquid is selectively formed on said substrate surface by forming a resist pattern on a part of said substrate surface in the flow channel, bringing said organic molecule into contact with said substrate surface whereon said resist pattern is formed, and then removing the resist pattern.

18. The method of manufacturing a device substrate according to claim 16, further comprising the step of forming said monolayer selectively on said substrate surface of the flow channel by forming the monolayer non-compatible with the liquid on said substrate surface in the flow channel, placing a photomask on the formed monolayer, irradiating the monolayer with high-energy ray, and then removing only the area not covered with the photomask.

19. The method of manufacturing a device substrate according to any one of claims 15 to 18, wherein said monolayer is formed by a chemical adsorption method.

20. The method of manufacturing a device substrate according to any one of claims 15 to 19, wherein:
said organic molecule has
(i) a functional group represented by the following General Formula (S4) as the terminal bonding functional group capable of forming a covalent bond with said substrate surface in the flow channel: 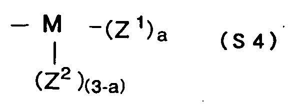 [in General Formula (S4), M is Si, Ti, Al or Sn; Z¹ represents at least one atom or atomic group selected from the group consisting of F, Cl, Br, I, -OH, -SCN, -NCO, and alkoxy group having 1 to 5 carbons; Z² represents at least one atom or atomic group selected from the group consisting of H, and alkyl group having 1 to 5 carbons; and a is an integer of 1 to 3],
(ii) a terminal group thereof not binding to the substrate being at least one characteristic group selected from the group consisting of methyl group, halogen-substituted methyl group, vinyl group, cyclic ether group having 2 to 4 carbons, phenyl group, halogen-substituted phenyl group, cyano group and the substituted derivatives thereof, and
(iii) a bivalent characteristic group represented by the following General Formula (S5) between said two terminal groups:
-C_{b}E_{2b}- (S5)
[in General Formula (S5), E represents at least one atom selected from the group consisting of H and F; and b is an integer of 2 to 22].

21. The method of manufacturing a device substrate according to claim 20, wherein said organic molecule has a structure wherein M represented by the General Formula (S4) is Si, and at least one bivalent characteristic group selected from the group consisting of the characteristic groups represented by the following General Formula (S6), -O-, -COO-, and -C₆H₄- and the substituted derivatives thereof is additionally bound between carbons constituting a carbon backbone of the bivalent characteristic group represented by General Formula (S5). 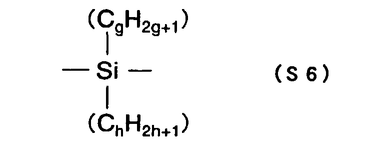 [in General Formula (S6), g and h each are independently an integer of 1 to 3.]

22. The method of manufacturing a device substrate according to any one of claims 15 to 19, wherein
the organic molecule has
(i) a functional group represented by the following General Formula (S7) as the terminal bonding functional group capable of forming the covalent bond with said substrate surface in the flow channel: 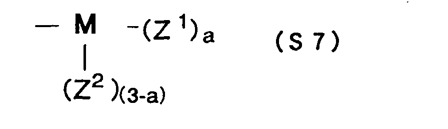 [in General Formula (S7), M is Si, Ti, Al or Sn; Z¹ is at least one atom or atomic group selected from the group consisting of F, Cl, Br, I, -OH, -SCN, -NCO, and alkoxy group having 1 to 5 carbons; Z² is at least one atom or atomic group selected from the group consisting of H and alkyl group having 1 to 5 carbons; and a is an integer of 1 to 3],
(ii) a terminal group thereof not binding to the substrate being at least one characteristic group selected from the group consisting of methyl group, halogen-substituted methyl group, vinyl group, cyclic ether group having 2 to 4 carbons, phenyl group, halogen-substituted phenyl group, cyano group and the substituted derivatives, and
(iii) a trivalent characteristic group represented by the following General Formula (S8) between the two terminal groups. 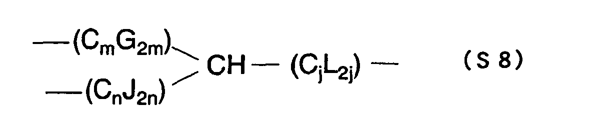 [in General Formula (S8), CⱼL₂ⱼ is a characteristic group binding to M above; CₘG₂ₘ and CₙJ₂ₙ each is a characteristic group binding to the terminal group at the terminal not binding to the substrate surface; G, J and L may be the same or different from each other, and represent at least one atom selected from the group consisting of H and F; j is an integer of 1 to 18; and m and n each are independently an integer of 0 to 7.]

23. The method of manufacturing a device substrate according to any one of claims 15 to 22, wherein the terminal bonding functional group of said organic molecule forming the covalent bond with said substrate surface in the flow channel is a halogenated silyl, alkoxy silyl, or isocyanate silyl groups, and the reaction of said terminal bonding functional group and said active hydrogen is a dehydrodeharogenation reaction, dealcoholization reaction or isocyanate-removing reaction.

24. The method of manufacturing a device substrate according to any one of claims 15 to 19, wherein said organic molecule is at least one organic molecule selected from the group consisting of the organic molecules represented by the following General Formulae (S20) to (S29) and the derivative thereof. 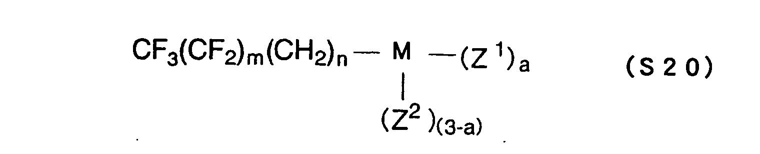 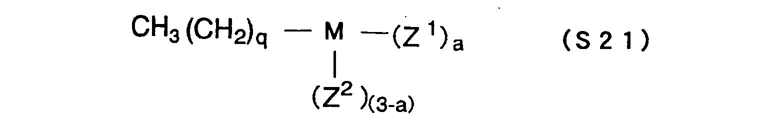 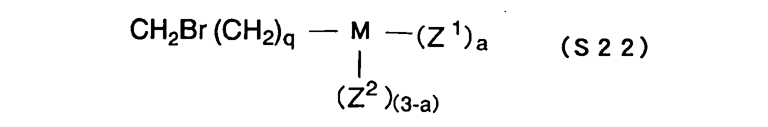 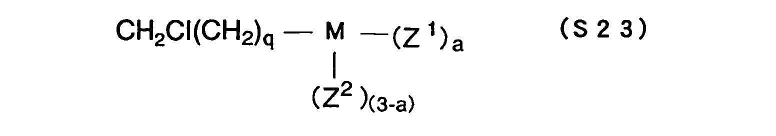 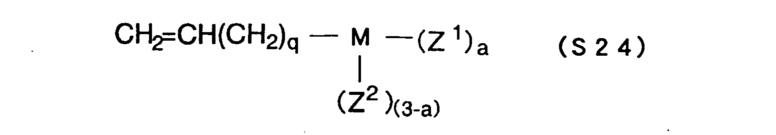 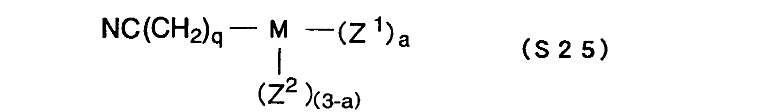 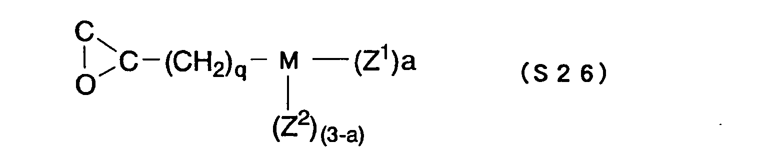 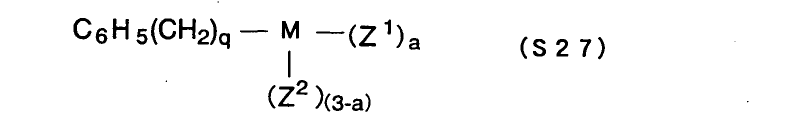 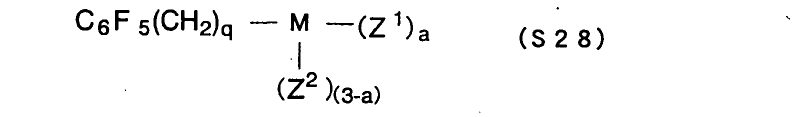 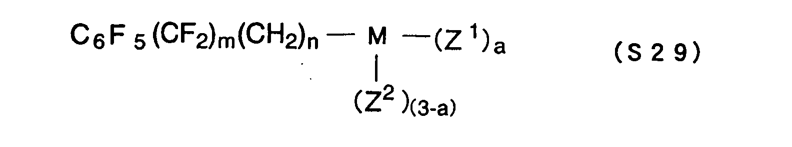 [in General Formulae (S20) to (S29), M is Si, Ti, Al or Sn; Z¹ represents at least one atom or atomic group selected from the group consisting of F, Cl, Br, I, -OH, -SCN, -NCO, and alkoxy group having 1 to 5 carbons; Z² represents at least one atom or atomic group selected from the group consisting of H and alkyl group having 1 to 5 carbons; a is an integer of 1 to 3; q is an integer of 2 to 22; m and n each are an integer satisfying the conditions represented by the following formulae (I) to (III) at the same time:
0 ≤ m ≤ 14 (I);
0 ≤ n ≤ 15 (II);
and
2 ≤ (m+n) ≤ 22 (III).]

25. The method of manufacturing a device substrate according to any one of claims 15 to 19, wherein said organic molecule is at least one organic molecule selected from the group consisting of organic molecules represented by the following General Formulae (S30) to (S39) and the derivatives thereof. 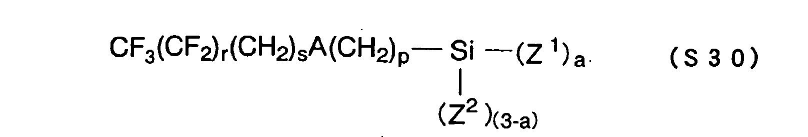 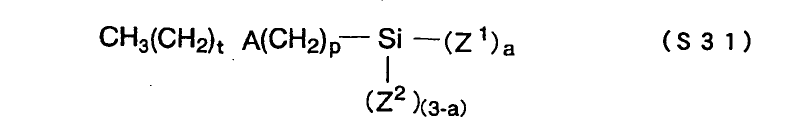 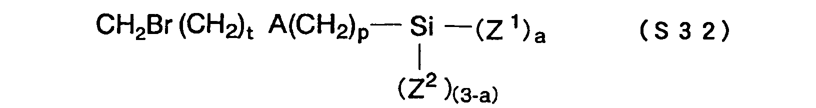 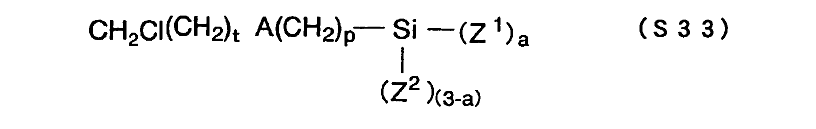 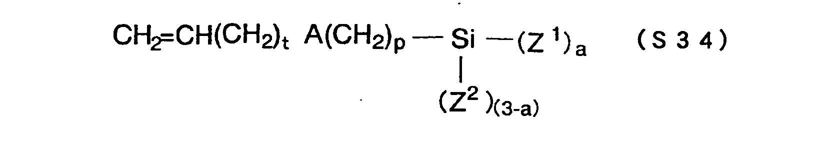 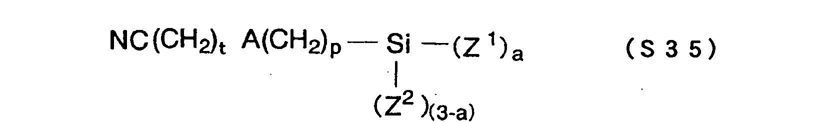 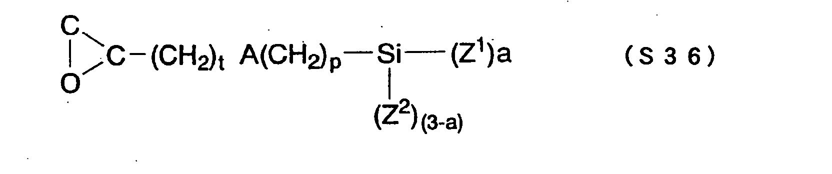 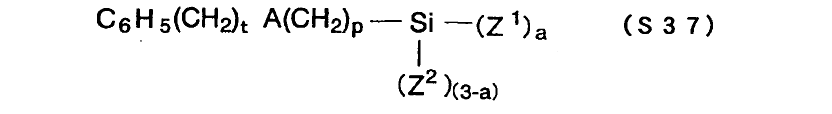 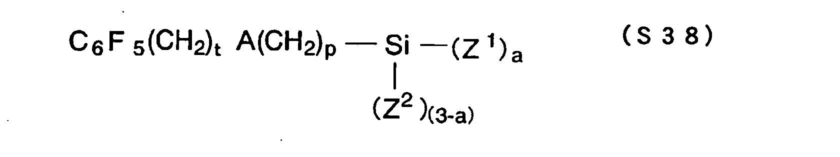 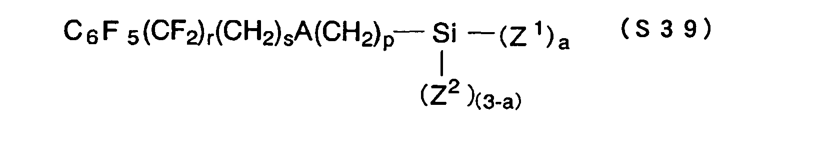 [in General Formulae (S30) to (S39), Z¹ represents at least one atom or atomic group selected from the group consisting of F, Cl, Br, I, -OH, -SCN, -NCO, and alkoxy group having 1 to 5 carbons; Z² represents at least one atom or atomic group selected from the group consisting of H, and alkyl group having 1 to 5 carbons; a is an integer of 1 to 3; A represents at least one bivalent characteristic group selected from the group consisting of the characteristic groups represented by the following General Formula (S9): 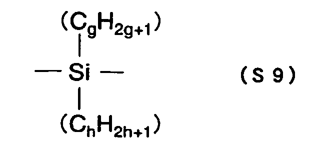 [in General Formula (S9), g and h each are independently an integer of 1 to 3.], -O-, -COO-, and -C₆H₄- and the substituted derivatives thereof; t is an integer of 1 to 10; p is an integer of 1 to 18; r and s each are an integer satisfying the conditions represented by the following formulae (IV) to (VI) at the same time:
0 ≤ r ≤ 14 (IV);
0 ≤ s ≤ 15 (V);
and
2 ≤ (r+s) ≤ 22 (VI).]

26. The method of manufacturing a device substrate according to any one of claims 15 to 19, wherein the organic molecule is at least one organic molecule selected from the group consisting of organic molecules represented by the following General Formulae (S40) to (S49) and the derivatives thereof. 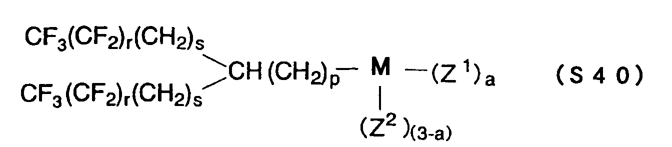 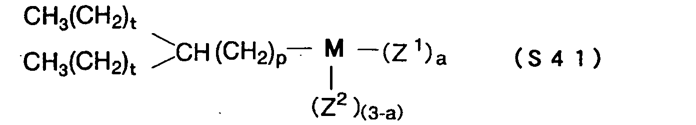 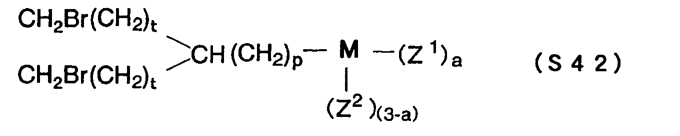 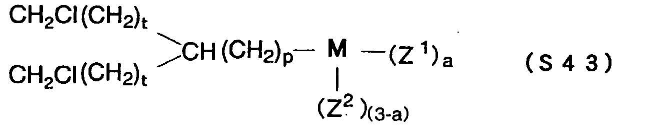 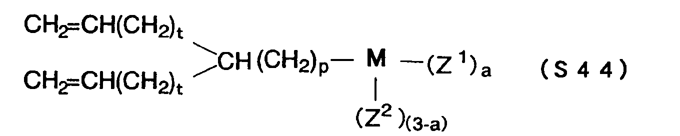 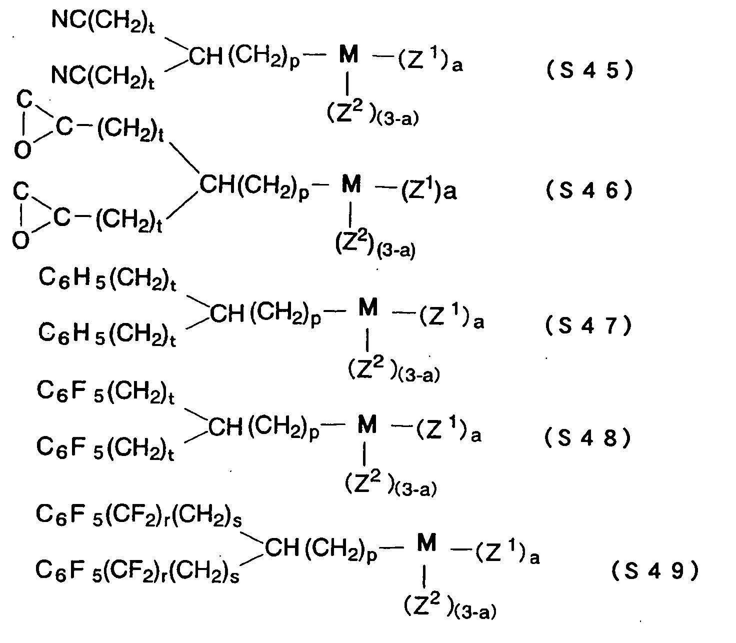 [in General Formulae (S40) to (S49), M is Si, Ti, Al or Sn; Z¹ represents at least one atom or atomic group selected from the group consisting of 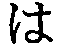 F, Cl, Br, I, -OH, -SCN, -NCO, and alkoxy group having 1 to 5 carbons; Z² represents at least one atom or atomic group selected from the group consisting of H, and alkyl group having 1 to 5 carbons; a is an integer of 1 to 3; t is an integer of 1 to 10; p is an integer of 1 to 18; r and s each are an integer satisfying the conditions represented by the following formulae (IV) to (VI) at the same time:
0 ≤ r ≤ 14 (IV);
0 ≤ s ≤ 15 (V);
and
2 ≤ (r+s) ≤ 22 (VI).]

27. The method of manufacturing a device substrate according to any one of claims 15 to 19, wherein said organic molecule is at least one selected from the group consisting of the following organic molecules.
CF₃(CF₂)₇(CH₂)₂SiCl₃
F(CF₂)₄(CH₂)₂O(CH₂)₁₅SiCl₃
CF₃COO(CH₂)₁₅SiCl₃
F(CF₂)₄(CH₂)₂Si(CH₃)₂(CH₂)₉SiCl₃
F(CF₂)₈Si(CH₃)₂(CH₂)₉SiCl₃
CF₃(CH₂)₂Si(CH₃)₂(CH₂)₁₅SiCl₃
CF₃CH₂O(CH₂)₁₅SiCl₃
CH₃(CH₂)₇(CH₂)₂SiCl₃
H(CH₂)₄(CH₂)₂O(CH₂)₁₅SiCl₃
CH₃COO(CH₂)₁₅SiCl₃
H(CH₂)₄(CH₂)₂Si(CH₃)₂(CH₂)₉SiCl₃
H(CH₂)₈Si(CH₃)₂(CH₂)₉SiCl₃
CH₃(CH₂)₂Si(CH₃)₂(CH₂)₁₅SiCl₃
CH₃CH₂O(CH₂)₁₅SiCl₃
CF₃(CF₂)₇(CH₂)₂TiCl(CH₃)₂
CF₃(CF₂)₇(CH₂)₂TiCl(C₃H₇)₂
CH₃(CH₂)₇AlCl(OC₂H₅)₂
CF₃(CF₂)₂(CH₂)₂Al(OC₂H₅)₃
CF₃(CF₂)₇(CH₂)₂Al(OCH₃)₃
CF₃(CH₂)₄SnCl(C₃H₇)₂

28. The method of manufacturing a device substrate according to any one of claims 15 to 27, wherein said substrate surface in the flow channel is provided with the active hydrogen by a surface treatment before said monolayer is formed.

29. The method of manufacturing a device substrate according to claim 28, wherein said surface treatment for providing the active hydrogen is high-energy ray irradiation.

30. A microchip comprising the device substrate according to any one of claims 1 to 13.

31. A microchip comprising the device substrate according to claims 1 to 13 and another substrate connected to the face of said device substrate whereon the flow channel is formed.

32. The microchip according to claim 31, wherein said monolayer non-compatible with the liquid is additionally formed on the connecting face of the other substrate connected.
